# EUROPEAN PATENT APPLICATION

(11) **EP 4 410 237 A1**
(43) Date of publication of application: **07.08.2024**
(21) Application number: 23305144.0
(22) Date of filing: 03.02.2023
(51) Int. Cl.: A61F 2/00

(54) **COMPOSITE IMPLANT INCLUDING A THREE-DIMENSIONAL MESH AND A FOAM AND METHODS ASSOCIATED THEREWITH**

(71) Applicant: Sofradim Production, 01600 Trevoux (FR)
(72) Inventor: GUERIN, Gaetan, 69005 LYON (FR); SERENI, Nicolas, 69100 VILLEURBANNE (FR)
(74) Representative: Germain Maureau

(57) **Abstract**

The present disclosure relates generally to composite implants configured for the repair or treatment of soft tissue, the composite implants including a three-dimensional mesh having one or more filaments and/or grip members and a compressible foam applied thereto. The three-dimensional implantable surgical mesh includes a first face and a second opposite face, and a three-dimensional dry foam attached to a plurality of filaments along the first face of the three-dimensional implantable surgical mesh.

## Description

### BACKGROUND

### Technical Field:

The present disclosure relates generally to composite implants including an implantable surgical mesh in a three-dimensional (e.g., non-planar) configuration and a foam positioned thereon, and in particular composite implants including a three-dimensional self-fixating mesh including one or more grip members and a foam positioned on at least the one or more grip members. The composite implants are suitable for the treatment or repair of soft tissue openings or wounds.

### Related Art:

Generally planar implantable surgical mesh including polymeric grips to ensure fixation directly to biological tissues or a different portion of a mesh, without using additional fixation, are known (e.g., see U.S. Patent No. 7,331,199). Surgical mesh free of grips and thermoformed into a generally three-dimensional configuration are also known (e.g., see U.S. Patent No. 10,675,137). Generally, without intending to be limited solely by theory, the thermoforming processes described, and the higher temperatures associated therewith, may damage the polymeric filaments and/or grips of the known generally planar mesh if thermoset into a three-dimensional configuration. Thus, filaments may be weakened and/or the grips will not be unable to attach directly to tissue or mesh.

Therefore, one objective of the present disclosure is to provide methods of forming and/or using implantable surgical mesh maintained in a three-dimensional configuration with a foam (and/or composite implants) to avoid damaging any filaments and/or grips. Another objective is to provide methods of forming and/or using three-dimensional self-fixating mesh (and/or composite implants) including grips configured to attach directly to tissue or a different portion of a mesh. Another objective includes providing three-dimensional self-fixating mesh (and/or composite implants) including grips configured to attach directly to tissue or a different portion of a mesh. The mesh may not be thermoformed or thermoset. In addition, since the grips remain configured to attach to tissue or other mesh, another objective includes controlling when and/or where the grips of the three-dimensional mesh (and/or composite implant) can actively attach to tissue or other portions of a mesh.

### SUMMARY

The present disclosure describes three-dimensional composite implants suitable for soft tissue repair, the composite implants include a three-dimensional implantable surgical mesh (with or without grip members extending therefrom) and a three-dimensional dry foam positioned on at least one face of the implantable surgical mesh. The dry foam being attached to a plurality of filaments of the mesh. Methods of forming the composite implants described herein are also provided.

In some embodiments, the three-dimensional implantable surgical mesh is a non-self-fixating mesh free of grip members and the foam is attached to a plurality of filaments that define at least a portion of the first face of the non-self-fixating mesh.

In some embodiments, the three-dimensional implantable surgical mesh is a self-fixating mesh including a plurality of grip members extending from a first face thereof and the foam is attached to the plurality of filaments that define the plurality of grip members.

In some embodiments, the present disclosure describes three-dimensional composite implants suitable for soft tissue repair, the composite implants include at least one mesh having grip members extending therefrom and a compressible foam positioned on at least a portion of the grip members. The composite implants described herein are configured to provide the medical staff or surgeon the ability to determine which portions or locations of the mesh or implant to activate the gripping ability of the grip members thereon, and/or those grip members not to activate, post-manufacturing of the implant. In addition, the composite implants described herein may be configured to determine the activation or inactivation of grip members of the composite implant, regardless or independent of the dissolution rate of the compressible foam.

In some embodiments, the foam may be a dry compressible foam configured to transition from a first foam configuration having a first thickness to a second foam configuration having a second thickness smaller than the first thickness upon the application of pressure thereto, without changing the non-planar configuration of the three-dimensional mesh.

In some embodiments, the portion of the first face of the three-dimensional implantable surgical mesh (with or without grip members) covered with the dry foam is configured to transition from the three-dimensional contour to a generally planar configuration when the foam is hydrated and/or absorbed.

In some embodiments, the three-dimensional composite implant is an implantable surgical mesh for hernia repair.

In some embodiments, the three-dimensional composite implant is an implantable surgical plug.

Methods of manufacturing a three-dimensional composite implant are also provided. The methods may include: combining a wet foam material with at least a first face, and particularly a plurality of filaments along the first face, of a generally planar implantable surgical mesh (with or without grip members) to form a wet foam-planar mesh composite; applying one or more molds to at least one side of the wet foam-planar mesh composite to transition the generally planar implantable surgical mesh into a three-dimensional implantable surgical mesh forming a wet foam-3D mesh composite; and drying the wet foam-3D mesh composite forming a dry foam-3D mesh composite implant. The one or more molds may be removed from the dry foam-3D mesh composite implant.

In some embodiments, combining the wet foam material with the generally planar implantable surgical mesh includes applying the wet foam material directly to a plurality of filaments along a first face of the generally planar implantable surgical mesh to form a layer of wet foam material thereon. The plurality of filaments may define the body of the mesh, the grip members, or both.

In some embodiments, applying one or more molds includes applying a first mold to a first side of the wet foam-planar mesh composite and further applying a second mold to a second opposite side of the wet foam-planar mesh composite. The first mold may be a solid mold and the second mold may be a porous mold e.g., the porous mold may include honeycomb-shaped pores. The mold(s) may be made of any suitable material including but not limited to metals, such as stainless steel, as well as polymeric materials, such as acrylonitrile butadiene styrene (ABS), polypropylene, or polylactic acid. In some embodiments, at least one of the molds may be a 3D printed mold.

In some embodiments, the methods of manufacturing a three-dimensional composite implant further include adding a strapping and a close piece along an outer edge of the mold, securing the mold to the wet foam-3D mesh composite with one or more clamps, or both.

In some embodiments, the methods of manufacturing a three-dimensional composite implant further include removing at least one, if not all, of the mold, the strapping, the close piece, or the one or more clamps, after drying.

In some embodiments, drying the wet foam-3D mesh composite includes drying the layer of wet foam material and the three-dimensional mesh in an oven.

In some embodiments, the methods of manufacturing a three-dimensional composite implant further include combining a frame assembly with the wet foam material prior to combining the wet foam material with the generally planar implantable surgical mesh, wherein the frame assembly includes a frame defining a frame cavity and a first sheet of inert material. The frame may be removed from the wet foam material prior to combining the wet foam material and the generally planar implantable surgical mesh.

In some embodiments, combining the frame assembly with the wet foam material includes casting, and optionally leveling, a layer of wet foam material inside the frame cavity and on top of the first sheet, and combining the wet foam material with the generally planar implantable surgical mesh includes applying the plurality of filaments and/or grip members positioned along the first face of the mesh into the layer of wet foam material.

In some embodiments, applying the plurality of filaments and/or grip members positioned along the first face of the mesh into the layer of wet foam material includes embedding the plurality of filaments and/or grip members into the wet foam by rolling a second face of the generally planar implantable surgical mesh opposite the first face with a roller.

In some embodiments, the methods of manufacturing a three-dimensional composite implant further include combining a frame assembly with the generally planar implantable surgical mesh prior to combining the wet foam material with the generally planar implantable surgical mesh, wherein the frame assembly includes a frame defining a frame cavity and a first sheet of inert material. The frame may be removed prior to applying the first mold to the wet foam-planar mesh composite.

In some embodiments, combining the frame assembly with the generally planar implantable surgical mesh includes positioning at least a portion of the generally planar implantable surgical mesh on the first sheet and within the frame cavity of the frame prior to combining the wet foam material with the generally planar implantable surgical mesh, and combining the wet foam material with the generally planar implantable surgical mesh includes casting a layer of wet foam material in the frame cavity and directly on the plurality of filaments and/or grip members positioned along the first face of the generally planar mesh.

In some embodiments, the methods described herein further include transitioning the three-dimensional implantable surgical mesh into the generally planar implantable surgical mesh as the dry foam is removed, e.g., hydrated and/or absorbed.

In some embodiments, the methods may include: combining a wet foam material with a plurality of grip members positioned on a first face of a generally planar self-fixating mesh to form a wet foam-planar mesh composite; applying a mold to at least one side of the wet foam-planar mesh composite transitioning the generally planar self-fixating mesh into a three-dimensional self-fixating mesh forming a wet foam-3D mesh composite; and drying the wet foam-3D mesh composite forming a dry foam-3D mesh composite implant. The mold may be removed from the dry foam-3D mesh composite implant.

Methods of soft tissue repair utilizing the three-dimensional composite implants described herein are also provided. In some embodiments, methods of soft tissue repair may include: inserting a three-dimensional composite implant into a site of implantation of a patient near a wound in the soft tissue in need of repair, the three-dimensional composite implant including a mesh having a first face and a second face opposite the first face, a plurality of grip members extending from the first face of the mesh, each of the plurality of grip members including a body extending between a first end attached to the mesh and a second end free of the mesh, and a compressible foam covering at least the second ends of the plurality of grip members, the compressible foam configured to transition from a first foam configuration having a first thickness to a second foam configuration having a second thickness smaller than the first thickness, and the grip members configured to transition from an inactive gripping configuration to a gripping configuration; deploying the three-dimensional composite implant across the wound in the soft tissue; applying pressure to a portion of the compressible foam to transition from the first foam configuration to the second foam configuration having the second thickness less than the first thickness thereby exposing the second ends of at least some portion of the plurality grip members; and transitioning the grip members from the inactive gripping configuration to the active gripping configuration to fixate the implant to the soft tissue.

### BRIEF DESCRIPTION OF THE DRAWINGS

The accompanying drawings, which are incorporated in and constitute a part of the specification, illustrate embodiments of the disclosure and, together with a general description of the disclosure given above, and the detailed description of the embodiments given below, serve to explain the principles of the disclosure.
FIG. 1 is a flowchart of methods of manufacturing a composite implant including a three-dimensional mesh and a foam as described in at least one embodiment herein;
FIG. 2 is a schematic side view of a generally planar self-fixating mesh including at least one grip member as described in at least one embodiment herein;
FIGS. 3A and 3B are schematic cross-sectional views of at least one grip member as described in at least one embodiment herein;
FIG. 4 is a schematic side view of forming a three-dimensional composite implant as described in at least one embodiment herein;
FIG. 5 is a flowchart of methods of manufacturing a composite implant including a three-dimensional mesh and a foam as described in at least one embodiment herein;
FIGS. 6A-6F are perspective views of forming a composite implant as described in at least one embodiment herein;
FIG. 7 is a schematic view of methods of combining grip members of a generally planar self-fixating mesh with a wet foam layer as described in at least one embodiment herein;
FIG. 8 is a schematic view of methods of combining grip members of a generally planar self-fixating mesh with a wet foam layer as described in at least one embodiment herein;
FIGS. 9A-9B are a perspective and side view, respectively, of a composite implant including a three-dimensional mesh and foam in a first configuration as described in at least one embodiment herein;
FIG. 10 is a side view of the composite implant of FIGS. 9A-9B including a three-dimensional mesh and foam in a second configuration as described in at least one embodiment herein;
FIGS. 11A-11B are a perspective and side view, respectively, of a composite implant including a three-dimensional mesh and foam as described in at least one embodiment herein;
FIG. 12 is a side view of a composite implant including a 3D non-self-fixating mesh and foam layer as described in at least one embodiment herein;
FIGS. 13A-13C are schematic views of a method of hydration and/or absorption of a composite implant as described in at least one embodiment herein.

### DETAILED DESCRIPTION

The present disclosure relates to composite implants including an implantable surgical mesh defining a three-dimensional (3D) configuration and a foam attached thereto. The foam may also define a three-dimensional configuration.

In some embodiments, the implantable surgical mesh includes a self-fixating mesh including one or more grip members. In some embodiments, the implantable surgical mesh includes a non-self-fixating mesh free of any grip members.

In some embodiments, the composite implant includes a self-fixating mesh. A self-fixating mesh includes one or more, and particularly a plurality, of grip members configured to grip directly to tissue or other overlapping portions of the same or different mesh. The foam covers at least a portion of the grip members, if not the entirety of the grip members. The foam may be a compressible foam configured to transition from a first configuration having a first initial thickness to a second configuration having a second subsequent thickness.

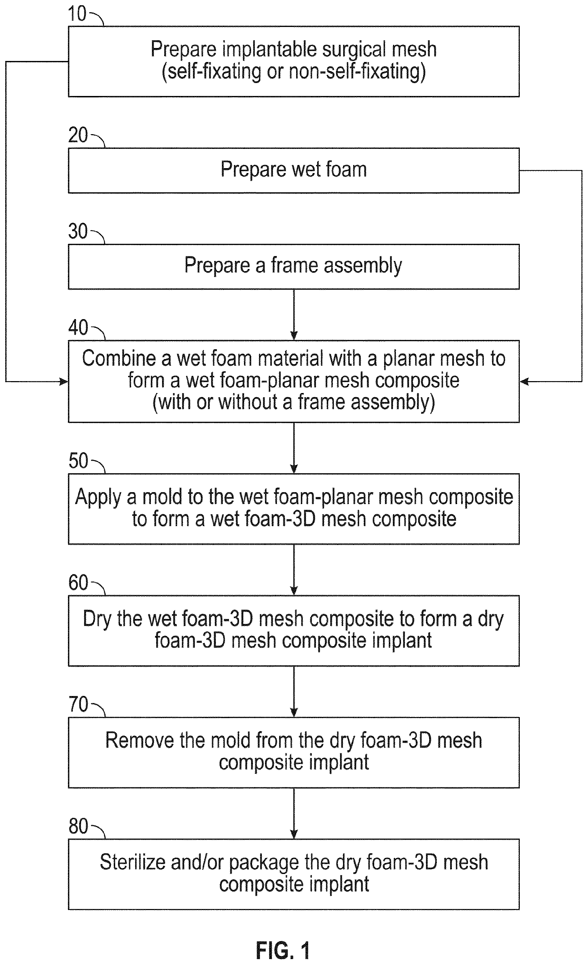

Methods of manufacturing a composite implant including a three-dimensional (3D) implantable surgical mesh (with or without grip members) and a foam are also provided herein. Generally, as shown in FIG. 1, the methods of manufacturing may include one or more of the following steps: preparing a planar implantable surgical mesh (e.g., self-fixating mesh and/or non-self-fixating mesh) 10; preparing a wet foam material 20; preparing a frame assembly 30; combining a wet foam material with a planar implantable surgical mesh forming a wet foam-planar mesh composite 40; applying one or more molds to at least one side of the wet foam-planar mesh composite forming a wet foam-3D mesh composite 50; drying the wet foam-3D mesh composite with the one or more molds positioned thereon forming a dry foam-3D mesh composite implant 60; and, removing the one or more molds from the dry foam-3D mesh composite implant 70. The dry foam-3D mesh composite implant may also be sterilized and/or packaged 80.

The initial steps of preparing a planar mesh 10, preparing of a wet foam material 20, and/or preparing a frame assembly 30 may be optional and/or may be performed in any order or combination.

### Planar Implantable Surgical Mesh Prep

As noted throughout the present disclosure, the composite implants described herein may include a three-dimensional (3D) implantable surgical mesh. In some embodiments, a three-dimensional (3D) implantable surgical mesh may be a three-dimensional (3D) self-fixating mesh, i.e., an implantable surgical mesh having a mesh body including one or more grip members extending therefrom wherein the mesh body defines a three-dimensional (3D) and/or non-planar configuration. However, to avoid damaging the grip members during manufacturing processes, the methods of manufacturing described herein begin with a planar self-fixating mesh (i.e., an implantable surgical mesh having a mesh body including one or more grip members extending therefrom wherein the mesh body defines a generally planar and/or singularly planar configuration) and transition the planar self-fixating mesh into a three-dimensional (3D) self-fixating mesh, particularly after being combined with a wet foam material as described herein.

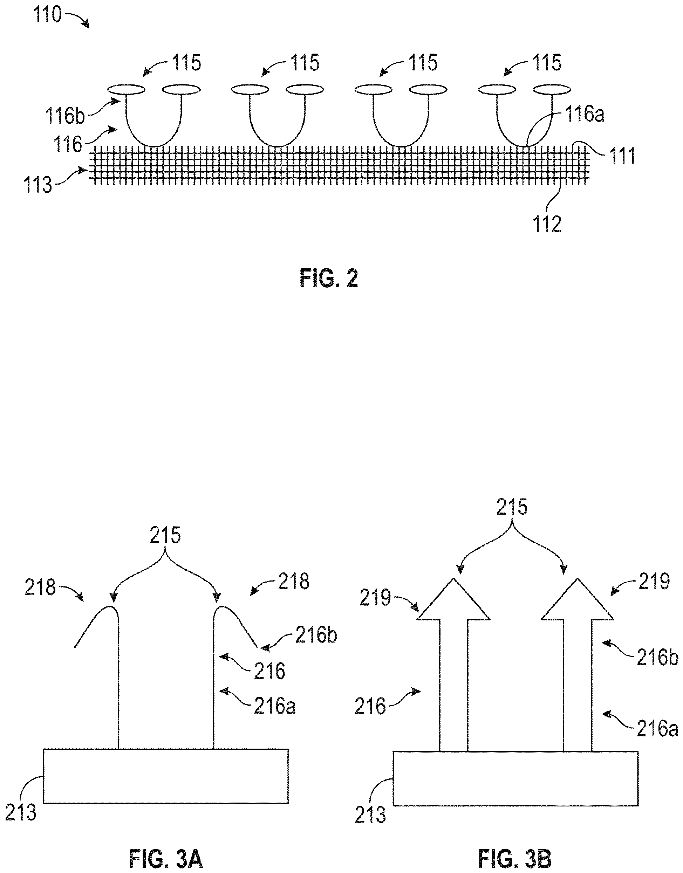

FIGS. 2-3B depict various planar self-fixating mesh. The planar self-fixating mesh 110 of FIG. 2 includes a mesh body 113 having a first face 111 and a second face 112 opposite the first face 111, at least the first face 111 including a plurality of grip members 115 extending therefrom. The grip members 115 are configured to attach directly to tissue, other grip members, and/or to other portions of the same or a different implantable mesh. Each grip member 115 includes a first end portion 116a of a grip body 116 attached to and/or extending from the mesh body 113 (and/or one of the mesh faces 111, 112). A second free end portion 116b of the grip body 116, opposite the first end portion 116a, extends farthest away from the mesh body 113 (and/or one of the mesh faces 111, 112) and may include a head portion 117 configured to grip to tissue and/or other portions of the implantable mesh 110 when overlapped, i.e., folded, rolled, etc.

As further shown in FIG. 2, in some embodiments, the one or more grip members 115 are spiked naps, wherein the head portion 117 is wider than the elongate body 116. Each spiked nap has a body 116 protruding from the first face 111 of the mesh body 113 and, at the second free end 116b of the body 116, a head 117 including a greater width than that of the body 116. The head 117 may be a generally spheroidal shape or a mushroom shape. The head portion 117 may include a smooth outer surface or may include a rough outer surface having asperities. The first end 116a of the body 116 of the spiked nap being interwoven with the filaments of the mesh body 113 of the planar implantable mesh 110.

The mesh 110 of FIG. 2 is one non-limiting example of a planar self-fixating mesh. The mesh 110 includes a mesh body 113 which is flat and/or generally defines a single plane, i.e., a self-fixating mesh in a planar configuration, a planar self-fixating mesh. The mesh body 113 of FIG. 2 does not yet define a three-dimensional (3D) and/or non-planar configuration, and as such is not a three-dimensional (3D) and/or non-planar self-fixating mesh as described herein. The mesh 110 of FIG. 2 also does not include a foam located on any portion thereof, particularly the grip members 115, and as such is not yet a composite or composite implant as described herein.

In some embodiments, a three-dimensional (3D) implantable surgical mesh may be a three-dimensional (3D) non-self-fixating mesh, i.e., an implantable surgical mesh free of grip members extending therefrom wherein the mesh body defines a three-dimensional (3D) and/or non-planar configuration.

The implantable surgical mesh, including the mesh body and optionally the one or more grip members, can be formed from one or more intertwined filaments and/or yarns. The filaments (and/or yarns) may be monofilaments or multi-filaments. The implantable mesh, and particularly the mesh body, may be formed using any suitable method known to those of ordinary skill including, but not limited to, weaving, knitting, braiding, crocheting, embroidering, and the like.

In some embodiments, the implantable mesh includes a knit mesh body. The knit mesh body may be knitted on a warp knitting machine, of the tricot or Raschel type, with at least two or three guide bars. In some embodiments, the planar implantable self-fixating mesh may be made or prepared as provided in any of the U.S. Patent Nos. 6,596,002; 7,331,199; 9,750,595; 9,186,235; 9,510,927, which are incorporated herein by reference.

The implantable mesh, and particularly the filaments that form the mesh, can be made from any biocompatible material, including bioabsorbable materials, non-bioabsorbable materials, and combinations thereof. The term "bioabsorbable" as used herein is defined to include both biodegradable and bioabsorbable materials. By bioabsorbable, it is meant that the materials decompose, or lose structural integrity under body conditions (e.g. enzymatic degradation or hydrolysis) or are broken down (physically or chemically) under physiologic conditions in the body such that the degradation products are excretable or absorbable by the body.

Representative natural bioabsorbable materials include, but are not limited to: polysaccharides, such as alginate, dextran, chitin, hyaluronic acid, cellulose, collagen, gelatin, fucans, glycosaminoglycans, and chemical derivatives thereof (substitutions and/or additions of chemical groups, for example, alkyl, alkylene, hydroxylations, oxidations, and other modifications routinely made by those skilled in the art); and proteins, such as albumin, casein, zein, silk, and copolymers and blends thereof, alone or in combination with synthetic polymers.

Synthetically modified natural polymers include, but are not limited to, cellulose derivatives, such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt.

Representative synthetic bioabsorbable polymers include, but are not limited to, polyhydroxy acids prepared from lactone monomers, such as glycolide, lactide, caprolactone, ε-caprolactone, valerolactone, and δ-valerolactone, as well as pluronics, carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s; polyalkylene oxalates; polyoxaesters; polyanhydrides; polyorthoesters; and copolymers, block copolymers, homopolymers, blends, and combinations thereof.

Some non-limiting examples of suitable non-bioabsorbable materials include polyolefins, such as polyethylene and polypropylene including atactic, isotactic, syndiotactic, and blends thereof; polyethylene glycols; polyethylene oxides; ultra-high molecular weight polyethylene; copolymers of polyethylene and polypropylene; polyisobutylene and ethylene-alpha olefin copolymers; fluorinated polyolefins, such as fluoroethylenes, including expanded polytetrafluoroethylene (ePTFE) and condensed polytetrafluoroethylene (cPTFE), fluoropropylenes, fluoroPEGSs, and polytetrafluoroethylene; polyamides, such as nylon and polycaprolactam; polyamines; polyimines; polyesters, such as polyethylene terephthalate and polybutylene terephthalate; aliphatic polyesters; polyethers; polyether-esters, such as polybutester; polytetramethylene ether glycol; 1,4-butanediol; polyurethanes; acrylic polymers and copolymers; modacrylics; vinyl halide polymers and copolymers, such as polyvinyl chloride; polyvinyl alcohols; polyvinyl ethers, such as polyvinyl methyl ether; polyvinylidene halides, such as polyvinylidene fluoride and polyvinylidene chloride; polyacrylonitrile; polyaryletherketones; polyvinyl ketones; polyvinyl aromatics, such as polystyrene; polyvinyl esters, such as polyvinyl acetate; copolymers of vinyl monomers with each other and olefins, such as etheylene-methyl methacrylate copolymers, acrylonitrile-styrene copolymers, ABS resins, and ethylene-vinyl acetate copolymers; alkyd resins; polycarbonates; polyoxymethylenes; polyphosphazine; polyimides; epoxy resins; aramids, rayon; rayon-triacetate; spandex; silicones; and combinations thereof.

In some embodiments, the implantable mesh is formed from a combination of bioabsorbable and non-bioabsorbable filaments. For example, a first set of non-bioabsorbable filaments may be used to form the mesh body and a second set of absorbable filaments may be used to form the grip members extending therefrom. Other various combination of absorbable and/or non-absorbable filaments may be used to form the mesh described herein.

In some embodiments, the self-fixating mesh may be selected from Parietex Progrip^{™} or Parietene Progrip^{™} (both commercially available from Medtronic). Parietex Progrip^{™} includes a planar mesh body made from one or more polyester filaments and grip members extending therefrom made from a bioabsorbable material such as poly lactic acid (PLA).

The grip-members of the self-fixating mesh may take any suitable form configured to attach directly to tissue and/or other portions of the implantable mesh (or a second implantable mesh). Some non-limiting suitable forms include spiked naps, hooks, barbs, and combinations thereof. For example, as shown in FIG. 3A, in some embodiments, the one or more grip members 215 may include a hook design. Each grip member 215 includes an elongate body 216 protruding from the planar mesh body 213 and, at the second free end 216b of the body 216, the head portion 217 includes a hook 218. The first end 216a of the body 216 being interwoven with the filaments of the planar mesh body 213 of the implantable mesh 210 and extending therefrom.

In another example, as shown in FIG. 3B, in some embodiments, the one or more grip members 215 may include a barb or piercing tip design. Each grip member 215 includes an elongate body 216 protruding from the mesh body 213 and, at the second free end 216b of the body 216, the head portion 217 includes a barb or piercing tip 219. The first end 216a of the body 216 being interwoven with the filaments of the mesh body 213 of the implantable mesh 210 and extending therefrom.

Although the grip members are schematically depicted as spike naps throughout the remaining Figures, the self-fixating mesh and/or composite implants including the self-fixating mesh described herein may include any suitable grip member configured to attach or grip to tissue and/or other portions of the implantable mesh (or a second implantable mesh).

The grip members, regardless of form, may be positioned on and/or extend from any portion of at least one face of an implantable mesh. The grip members, like the mesh body, may be formed from one or more filaments which may be made from any of the bioabsorbable and/or non-bioabsorbable materials described hereinabove. In some embodiments, the grip members may be made from a bioabsorbable material, i.e., polylactic acid (PLA) or copolymers of PLA and trimethylene carbonate (TMC).

In some embodiments, the length of each of the grip members, measured from the face of the mesh body from which the grip members project from, to the end of the second free end of the grip member, is smaller than the thickness of the implantable mesh body in which the grip members may penetrate and become entangled thereto. In some embodiments, the length of each of the grip members can be between about 0.1 and 5 millimeters. In some embodiments, the length of each of the grip members can be between about 0.5 and 3.5 millimeters. In some embodiments, the length of each of the grip members can be between about 1 and 3 millimeters. In some embodiments, the length of each of the grip members can be between about 1.5 and 2.5 millimeters.

### Wet Foam Material Prep

As further provided in FIG. 1, prior to being combined with a frame assembly and/or a planar implantable surgical, and particularly the grip members of a self-fixating mesh, a wet foam material may be prepared including forming a solution of containing a polymeric foaming agent; and foaming the solution into a wet foam material.

Forming the solution includes mixing at least one biocompatible polymeric foaming agent, which is soluble within physiological conditions, with a pharmaceutical carrier to form a solution. Some non-limiting examples of suitable pharmaceutical carriers include water, saline, dextrose solution, buffer solution, etc. One or more biocompatible plasticizers and/or one or more biocompatible additives, each of which are also soluble within physiological conditions or similar fluids such as saline, may also be added to the solution. In addition, the solution may further include one or more bioactive agents.

Some non-limiting examples of suitable biocompatible polymeric foaming agents include proteins such as gelatin, collagen, poloxamers, polysorbates, methylcellulose (MC), hydroxypropyl methylcellulose (HPMC), hydroxypropyl cellulose (HPC), hydroxyethyl cellulose (HEC) and mixtures thereof. In some embodiments, the biocompatible polymeric foaming agent is collagen and/or collagen based.

In some embodiments, the foaming agent is hydroxypropyl methylcellulose. The hydroxypropyl methylcellulose may have a methyl substitution degree (DS_{methoxy}) ranging from 1 to 2.5 or 1.5 to 2. The hydroxypropyl methylcellulose may have a hydroxypropyl molar substitution (MS_{hydroxypropoxyl}) ranging from 0.1 to 1 or 0.2 to 0.5. In some embodiments, the hydroxypropyl methylcellulose may be combined with water to form an aqueous solution. The dynamic viscosity of the hydroxypropyl methylcellulose may represent from 1 to 100mPa.s⁻¹; or 3 to 50mPa.s⁻¹, measured as a 2 weight % aqueous solution at 20°C using an Ubbelohde-type viscometer.

In some embodiments, the foaming agent is one or more collagen compounds. The expression "collagen compound" is understood in the sense of the present application to mean collagen which has least partially lost its helical structure by heating or any other method, or gelatin. The term "gelatin" here includes commercial gelatin made from collagen which has been at least partially hydrolyzed and which has a molecular weight of less than 100kDa. The collagen compound used may be formed from hydrolyzed collagen, composed of a chain and having a molecular weight close to around 100 kDa. In the context of the present disclosure, the expression "a chains" is understood to mean intact a chains or fragments produced by the loss of a small number of amino acids. The term "unhydrolyzed" is understood here to mean that less than 10% of the collagen chains have a molecular weight of less than around 100 kDa. If heating is used to denature the helical structure of the collagen, the heating must be moderate and carried out under conditions arranged in order to avoid the degradation, by hydrolytic cleavage, of the gelatin thus formed. The collagen possibly being used in the context of the present application may be of animal origin, such as for example type I porcine or bovine collagens. Native collagen may be used, in acid solution or after conversion, in order to eliminate the telopeptides by pepsin digestion.

The at least one biocompatible polymeric foaming agent may represent from: 0.01 to 25 weight percent; 0.1 to 20 weight percent; 0.5 to 15 weight percent; or 1 to 10 weight percent, of the solution.

Some non-limiting examples of suitable carriers and/or solvents include water (e.g., sterile water, non-sterile water, distilled water, spring water, etc.), saline, dextrose solution, and the like. In some embodiments, the solvent is sterile water and the solution is an aqueous solution.

Some suitable non-limiting examples of one or more plasticizers include polyhydric alcohols such as glycerol, sorbitol, xylitol, mannitol, propylene glycol, polyethylene glycol, and mixtures thereof. In some embodiments, the solution may include a plasticizer such as glycerol, sorbitol, or both. When incorporated into the solution, the plasticizer may represent from: 0.5 to 40 weight percent; 1 to 20 weight percent; or 2 to 10 weight percent, of the solution.

Some suitable non-limiting examples of the one or more biocompatible additives include alginates, pectins, carrageenans, cellulose based derivatives such as cellulose ethers such as methylcellulose (MC), hydroxypropyl methylcellulose (HPMC), hydroxypropyl cellulose (HPC), hydroxyethyl cellulose (HEC), polysaccharides gums such as xanthan gum and guar gum, dextrans, hyaluronans and hyaluronate salts, pullulans, maltodextrins, gelatin, polyvinyl-alcohol and polyethylene-glycol, and mixtures thereof. In some embodiments, the solution may include a biocompatible additive such as sodium hyaluronate. The sodium hyaluronate may have an intrinsic viscosity at 25°C ranging from 0.3 to 3.0m³/kg. When incorporated into the solution, the additive may represent from: 0.1 to 10 weight percent, 0.2 to 5 weight percent, or 0.5 to 3 percent of the solution.

Bioactive agents include substances which are beneficial and tend to promote the healing process. Suitable examples of bioactive agents include, for example, biocidal agents, antimicrobial agents, antibiotics, anti-proliferatives, medicants, growth factors, anti-clotting agents, clotting agents, analgesics, anesthetics, anti-inflammatory agents, wound repair agents and the like, chemotherapeutics, biologics, protein therapeutics, monoclonal or polyclonal antibodies, DNA, RNA, peptides, polysaccharides, lectins, lipids, probiotics, diagnostic agents, angiogenics, antiangiogenic drugs, polymeric drugs, and combinations thereof. In some embodiments, the bioactive agent may represent less than 10% and particularly less than 5% of the solution.

The mixture of at least the foaming agent and the carrier can be heated up to 60°C, particularly between 20°C and 50°C, more particularly about 40°C, during mixing to form a solution.

In some embodiments, the wet foam may include one or more of hyaluronate salts, HPMC, glycerol, and combinations thereof.

In some embodiments, the wet foam may include collagen compounds, glycerol, and combination thereof.

After forming the solution including the polymeric foaming agent, and optionally at least one of the plasticizer or additives, in the carrier, the solution may be further processed into a wet foam material (step 24). Foaming may be conducted via air being mechanically or physically entrained into the solution to form a wet foam material including a first discontinuous air phase, and a second continuous aqueous phase. The lamella or fluid film of the air bubbles entrained within the wet foam material is viscous due to the presence of the polymeric foaming agent. The flow of any liquid retained in the lamella of the air bubbles is minimized, reduced, or prevented in the obtained wet foam material and as such, the wet foam material can be described as elastic and not able to flow. This wet foam material may have a density ranging from about 0.05 g.cm⁻³ to 0.50 g.cm⁻³; 0.10 g.cm⁻³ to 0.40 g.cm⁻³; or 0.20 g.cm⁻³ to 0.30 g.cm⁻³.

Advantageously, one or more of these characteristics of the wet foam material offers the possibility to cover the grip members, in various shapes, designs, or thicknesses, using a variety of different manners including but not limited to at least one of casting, molding, rolling, screening, dropping with a utensil such as a syringe, spoon, spatula, brush, sponge, roller, and the like. For example, it is possible to cover (or not cover) all or only selected portions of a face of the implantable self-fixating mesh with the wet foam material and/or dry compressible foam.

In some embodiments, the foaming step may include heating the solution to between 20°C and 60 °C followed by mechanical agitation, such as via whisking or mixing using rotor-stator technology, whisking of the warmed solution to form the wet foam material. For example, the heated solution may be sheared by mechanical agitation to incorporate air into the solution to produce a wet foam material. The wet foam may then be combined with the planar mesh and/or a frame assembly as described in more detail hereinbelow.

### Frame Assembly Prep

Prior to formation of the composite implants described herein, in some embodiments, a frame assembly may be prepared by combining an inert and flexible sheet with a rigid frame on top of the inert upper sheet.

In the present application, a sheet is said to be inert if it does not react with compounds of the wet foam of materials described above for the preparation of the solution. The sheet may be made of any suitable non-stick and/or inert material such as for example, non-limitingly, polypropylene (PP), polytetrafluoroethylene (PTFE), silicon, or a material which may be easily separated from the fabric after drying. In some embodiments, the sheet may be made of silicon.

A rigid frame can be added on top of the sheet, each frame defining a frame cavity and each sheet defining a surface area being larger than or equal to a surface area defined by the frame cavity. The rigid frame may be made of any suitable material such as for example, non-limitingly, polypropylene (PP), polytetrafluoroethylene (PTFE), silicon, polyethylene terephthalate glycol (PTEg), polyethylene (PE), Acrylonitrile butadiene styrene (ABS), aluminum, and stainless steel. In some embodiments, the frame may be made of stainless steel.

### Composite Implant Formation

As further provided in FIG. 1, once the mesh, the wet foam, and/or the frame assembly are ready to be combined, the composite implants described herein can be formed. More particularly, methods of forming and/or manufacturing a three-dimensional composite implant include: combining a planar implantable surgical mesh and a wet foam to form a wet foam-planar mesh composite, with or without a frame assembly (step 40); applying a mold to the wet foam-planar mesh composite to form a wet foam-3D mesh composite (step 50); drying the wet foam-3D mesh composite to form a dry foam-3D mesh composite implant (step 60); removing the mold from the dry-foam-3D mesh composite implant (step 70); and sterilizing and/or packaging the dry foam-3D mesh composite implant (step 80).

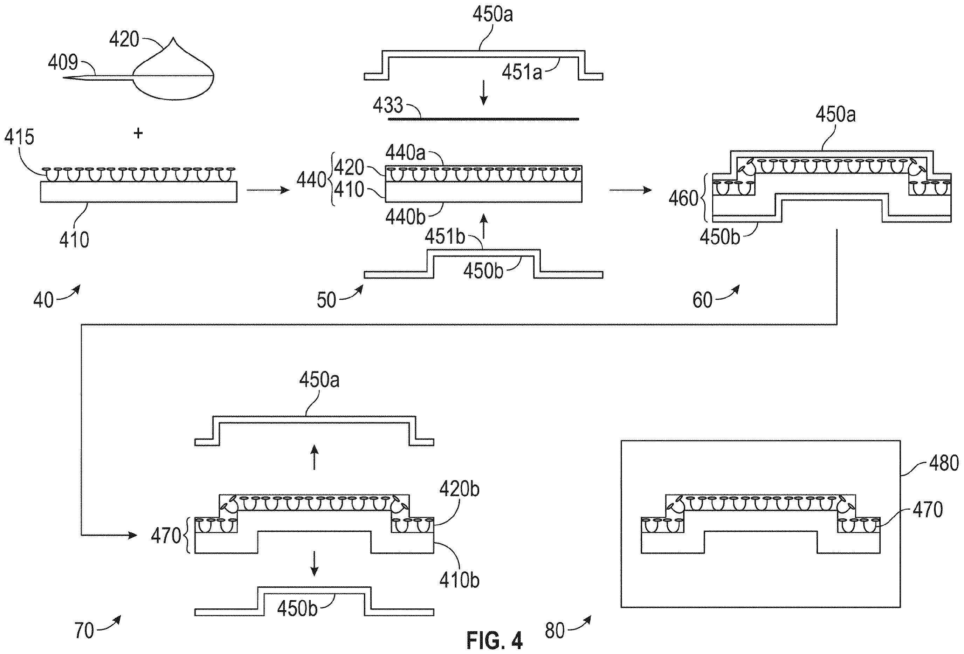

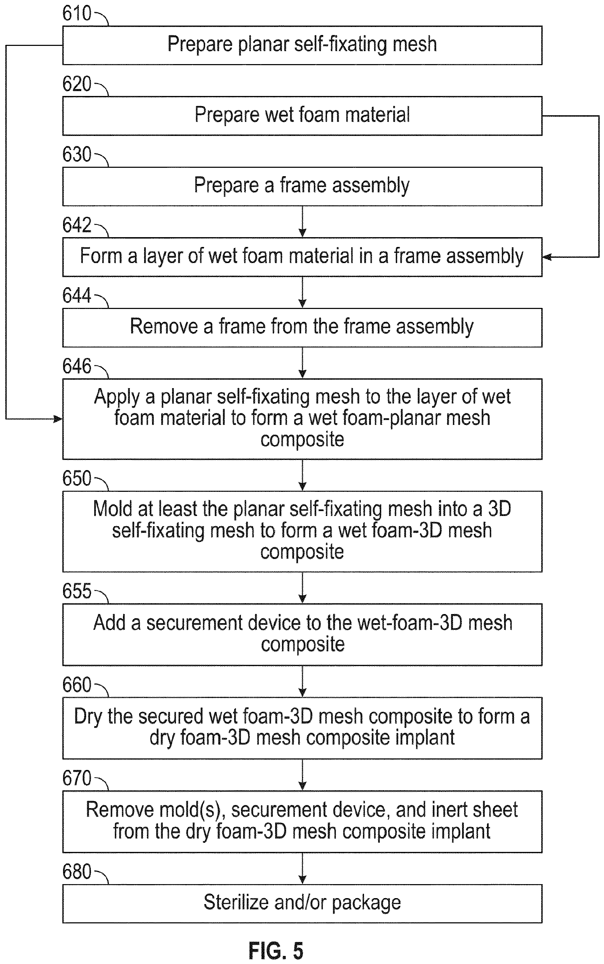

The wet foam material and the planar mesh may be combined (step 40) in any suitable manner. In some embodiments, the wet foam material and the planar mesh may be combined without the aid of a frame assembly to form a composite implant as described herein (FIG. 4). In some embodiments, the wet foam material and the planar mesh may be combined with the aid of a frame assembly to form a composite implant as described herein (FIGS. 5-8). The frame assembly may be combined with the wet foam before or after the wet foam is combined with and/or cast on the planar implantable surgical mesh.

In some embodiments, as shown in FIG. 4, at step 40, the wet foam material 420 may be applied directly to the grip members 415 of the planar self-fixating mesh 410 (without a frame assembly) to form a wet foam-planar mesh composite 440. Due to the high viscosity properties of the wet foam material 420, the wet foam material 420 can be applied directly to the mesh 410 to form a layer of wet foam material thereon. The layer may extend completely across the mesh or in any variety of planar patterns including but not limited to, stripes, polygons, circles, ovals, rings, dots, dashes, arrows, numbers, letters, tear drops, stars, etc. The wet foam material 420 can be directly applied to grip members 415 of the mesh 410 using any suitable utensil 409, including but not limited to a syringe, spoon, fork, ladle, scoop, straw, brush, roller, and/or piping bag.

As further depicted in FIG. 4, at step 50, a first and second mold 450a, 450b may be added to the opposite faces 440a, 440b of the wet foam-planar mesh composite 440 to transition at least the planar self-fixating mesh 410 into a three-dimensional (3D) and/or non-planar configuration thereby forming a wet foam-3D mesh composite 460 between the molds 450a, 450b. The layer of wet foam material 420 may also be molded into a three-dimensional wet foam material 420. The mold(s) 450a, 450b temporarily alter the configuration of the mesh 410 and the wet foam material 420 by applying mechanical pressure thereto. However, in most embodiments, the mold(s) 450a, 450b do not permanently altar, i.e., thermoset, the mesh in a 3D configuration. For example, if the molds are removed from the wet foam-3D mesh composite prior to drying, the mesh, alone or with the wet foam, may generally return to the prior planar configuration.

At least one, if not both, of the first and second molds 450a, 450b define a generally non-planar and/or three dimensional inner surface 451a, 451b. In some embodiments, the inner surface 451a, 451b of at least one of the molds 450a, 450b may also include and/or be formed of a non-stick material to avoid sticking to either layer 410, 420 of the wet foam-planar mesh composite 440. In some embodiments, an inert sheet 433 may be added to the outer surface of at least the layer of wet foam material 420 along the face 440a of the wet foam-planar mesh composite 440 prior to adding the mold 450a. In some embodiments, at least one mold, if not both, may not be in complete or continuous contact with the entire wet foam to allow air circulation thereto to dry the wet foam material.

As further depicted in FIG. 4, at step 60, the wet foam-3D mesh composite 460, including the one or more molds 450a, 450b, may be dried transitioning the wet foam material 420 into a dry foam 420b and forming a dry foam-3D mesh composite implant 470. The dry foam 420b may be a dry compressible foam. The drying process may involve exposing the wet foam 3D mesh composite 460 to adapted conditions (temperature, relative humidity, air flow) to form the dry foam. However, these environmental conditions may not be sufficient to damage the grip-members of the mesh and/or thermoset the self-fixating mesh in the non-planar and/or 3D configuration.

After drying, at step 70, the molds 450a, 450b, (and any inert sheets 433) may be removed from the dry foam-3D mesh composite implant 470. In the dry format, the layer of dry foam 420b holds the self-fixating mesh 410, via at least the grip members 415 embedded in the dry foam 420b, in the non-planar or 3D configuration 410b.

Once formed, the dry foam-3D mesh composite implant 470 (composite implant) may be sterilized and/or packaged (step 80) using a suitable manner known to those skilled in the art.

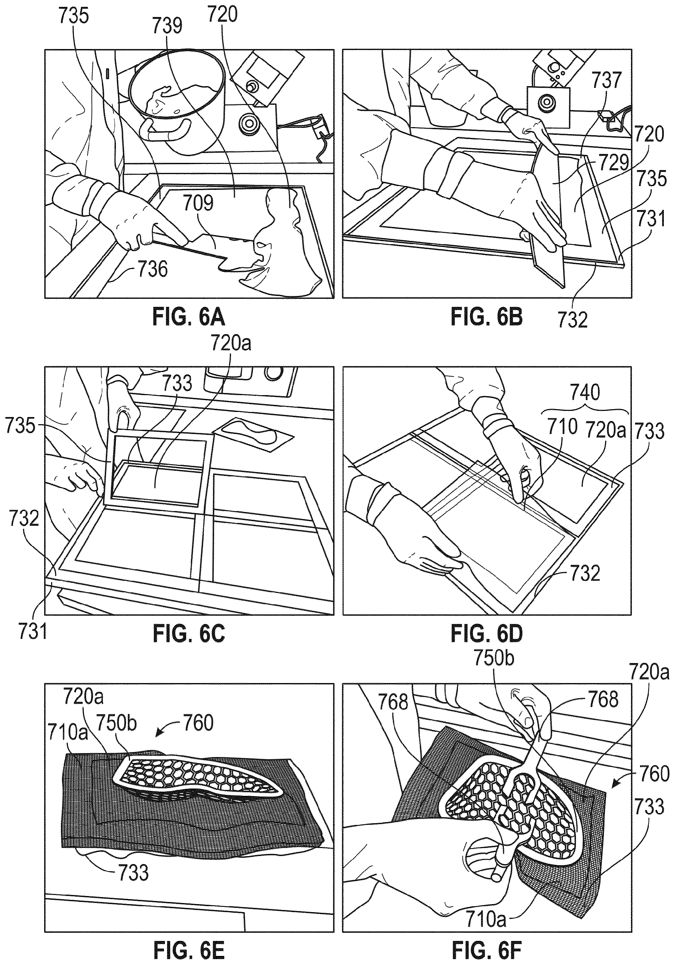

As depicted in FIGS. 5-6F, in some embodiments, the wet foam material and a planar implantable surgical mesh, particularly a self-fixating mesh, may be combined with the aid of a frame assembly to form a composite implant. More particularly, methods of forming and/or manufacturing a composite implant as described herein may include: preliminarily preparing or providing a planar self-fixating mesh (step 610), a wet foam material (step 620) and/or a frame assembly (step 630) as similarly described herein (steps 10, 20, 30, respectively, of FIG. 1); forming a layer of wet foam material in a frame assembly (step 642) (FIGS. 6A-6B); removing a frame from the wet foam layer (step 644) (FIG. 6C); applying the planar self-fixating mesh, and particularly the grip members of a planar self-fixating mesh, into the wet non-flow foam layer forming a wet foam-planar mesh composite (step 646) (FIG. 6D); positioning at least one or more molds onto an exterior of the wet foam-planar mesh composite to transition the generally planar mesh into a mesh defining a three-dimensional configuration to form a wet foam-3D mesh composite (step 650) (FIG. 6E); adding a securement device to the wet foam-3D mesh composite securing the one or more molds thereto (step 655) (FIG. 6F); drying the wet-foam-3D mesh composite including the one or more molds and any securement devices to transition the wet foam material into a dry compressible foam positioned on the grip members of the non-planar mesh to form a dry composite implant (e.g., a non-planar or three-dimensional composite implant) including a three-dimensional mesh and a dry compressible foam (step 660); and removing, from the dry composite implant, the one or more molds, any securement devices, and/or any inert sheets of the frame assembly still attached thereto (step 670). The dry composite implant may be further sterilized and packaged for distribution as needed (step 680).

FIG. 6A depicts a wet foam material 720 being applied inside a cavity 736 of one or more frames 735 (step 642) . For example, one or more dollops of the wet foam material 720 can be placed directly on top of one of the sheets 739 upon which one of the frames 735 is secured and within the cavity 736 defined by the frame 735. In some embodiments, a utensil 709, such as a spatula, ladle, scraper, or spoon, may be used to apply the wet foam material 720. The volume of wet foam 720 applied to a single frame 735 may be larger than the volume of the single respective cavity 736 defined by the single frame 735. The glass plate 731, upon which the base sheet 732, the upper sheet 733, and the frame 735 may be positioned, defines a level or planar surface.

FIG. 6B depicts casting and/or leveling the wet foam material 720 being spread and/or leveled across the cavity 736 and along the top of the frame 735 to define a generally planar wet foam layer 720a of a consistent thickness generally equal to the thickness of the frame 735 and/or cavity 736. For example, the spreading, casting and/or leveling of the wet foam material 720 may be performed with a smoothing device 729, such as a scraper or screed, being pulled across the top of the frame(s) 735 to level the foam material 720 with the top surface of the frame 735.

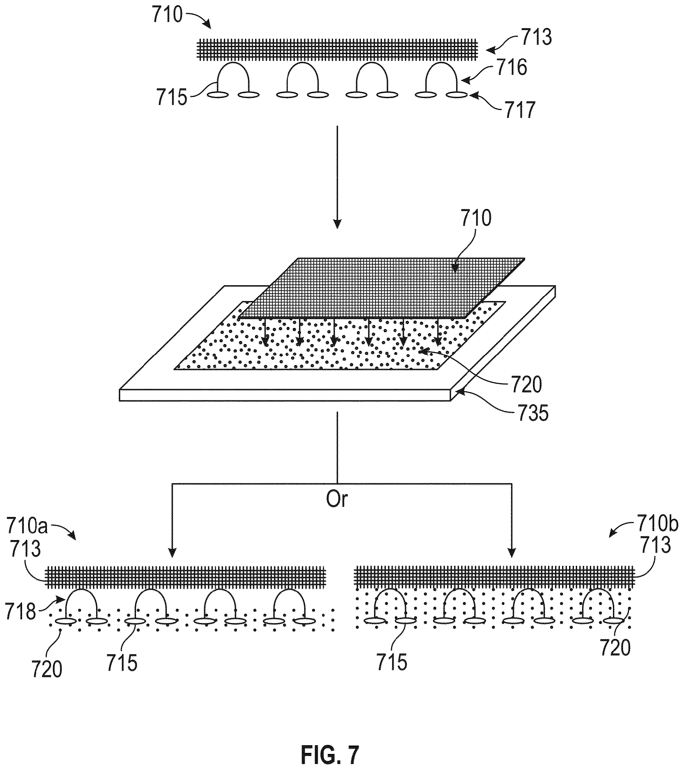

Because the wet foam material 720 can be leveled to the top of the frame 735, the thickness of the frame 735 can be used to define the thickness of the wet foam layer 720a and as result, the thickness of the dry compressible foam 720b, as schematically depicted in FIG. 7. For example, when a frame 735 thickness is less than the length of the body 716 of the grip members 715, the wet foam layer 720a can be spread, cast and/or leveled to fill the entire frame 735 so that the grip members 715 will be only superficially covered thereby creating a gap 718 between the mesh body 713 and the wet foam layer 720a (and/or the compressible foam 720b when dried), as shown in the composite to the bottom left 710a of FIG. 7. In another example, when the frame 735 thickness is greater than the length of the body 716 of the grip members 715, the wet foam layer 720a can be cast and leveled to fill the entire frame 735 so that the grip members 715 will be fully covered and the mesh body 713 and the wet foam layer 720a (and/or the compressible foam 720b when dried) may abut up against each other, as shown in the composite to the bottom right 710b of FIG. 7.

In some embodiments, following the spreading, casting and/or leveling of the wet foam material 720 to form a wet foam layer 720a, the frame 735 may be removed prior to combining the wet foam and the mesh. As depicted in FIG. 6C, at step 644, one or more frames 735 can be removed from the wet foam layer 720a, leaving the wet foam layer 720a positioned on the removable sheet 733 and/or the base plate 731. Particularly, in some embodiments, the methods of manufacturing described herein may include removing any frame fasteners 737 from the frame(s) 735 and then removing the frame(s) 735 from the layer 720a of wet foam material 720.

After removal of the frame(s) 735, as shown in FIG. 6D, at step 646, a generally planar self-fixating mesh 710 may be lowered onto the top surface of the wet foam layer 720a with the grip members (not visible in FIG. 6D) facing downward and leading the way towards the layer 720a of the wet foam 720 with the mesh body trailing behind the grip members 715 forming a wet foam-planar mesh composite 740. The mesh body may or may not enter the wet layer 720a. The top surface of the foam layer is opposite the bottom surface of the foam layer which sits on a top face of the inert sheet.

In some embodiments, after being positioned in the wet foam layer, the generally planar mesh may be rolled upon with a roller to ensure the grip members embed into the foam layer. For example, the roller may be rolled across the second surface of the mesh which is opposite the first surface including the wet foam and/or the grip members.

As depicted in FIG. 6E, the inert sheet 733 covered with the wet foam-planar mesh composite 740 can be separated from the base plate 731 and combined with a pair of molds 750a , 750b to transform the planar mesh 710 into a three-dimensional mesh 710a and form a wet foam-3D mesh composite 760. In FIG. 6E, the first mold 750a is hidden beneath the wet foam-3D mesh composite 760 and in embodiments may be a solid mold. The second mold 750b is shown positioned on and pressed into the second face 712 of the mesh 710 opposite the grip members to transition the generally planar mesh 710 into a non-planar configuration, i.e., a three-dimensional mesh 710a, while the wet foam material 720 (and/or wet foam layer 720a) remains positioned on the mesh 710a in a wet form.

The first or second mold can be any suitable mold which renders the mesh non-planar, alone or with the foam layer, without damaging the grip members or the foam layer. For example, the first or second mold may be solid mold including a porous mold, such as a mold including honeycomb-shaped pores. One example of a porous mold is shown in FIGS. 6E and 6F. The molds may be made of any suitable material such as for example, non-limitingly, polypropylene (PP), polytetrafluoroethylene (PTFE), polyethylene terephthalate glycol (PTEg), polyethylene (PE), Acrylonitrile butadiene styrene (ABS), aluminum, and stainless steel. In some embodiments, the frame may be made of Acrylonitrile butadiene styrene (ABS).

In some embodiments, at least one of the molds is a solid mold configured to shape a self-fixating mesh into a three-dimensional shape or contour similar to a Dextile Mesh ^{®} (Medtronic) (e.g., see U.S. Patent No. 10,675,137).

In some embodiments, the first and second molds are both solid molds. In other embodiments, the first and second molds are both porous molds. In still other embodiments, the first mold is a porous mold and the second mold is a solid mold. For example, in some embodiments, a porous mold including honey-combed shaped pores may be applied to the second face of the mesh and a solid mold may be applied to the bottom face of the inert sheet. It is envisioned that use of a solid mold on the inert sheet may produce a dry foam having a smooth outer surface. It is further envisioned that use of a porous mold on the inert sheet may allow portions of the inert sheet extending across the openings in the pores to seep into the openings, along with some portion of the wet foam material, thereby producing a dry foam having a roughened or textured outer surface.

In order to not damage the grip members of a self-fixating mesh (and/or the filaments of the non-self-fixating mesh), which may be protected by being embedded in the wet foam layer 720a, molding of the mesh can be performed without heat or with heat at a temperature sufficiently low enough not to damage or alter the grip members (and their ability to grip to tissue or mesh), the mesh, or the foam. In addition, unlike thermoforming, the molding of the implantable surgical mesh described herein does not permanently change the configuration of the mesh. Rather, the molding transforms the planar mesh into a non-planar configuration and the drying of the foam retains the mesh in the non-planar configuration. As a result, in some embodiments, when the dry foam is removed from the non-planar mesh, the mesh may naturally return to a generally planar configuration. By not being thermoset or permanently altered, the implantable mesh maintains the ability and/or flexibility to adapt to new tissue growth surrounding the mesh following implantation.

FIG. 6F depicts securing one or more molds 750a, 750b, to the wet foam-3D mesh composite 760. As shown, in some embodiments, the first or second molds 750a, 750b can be secured to the wet foam-3D mesh composite 760 via one or more securement devices 768, such as clamps or clips, to retain the non-planar configuration of the mesh 710a, alone or with the wet foam layer 720a, within the one or more molds 750a, 750b.

After the wet non-flow foam layer 720a and the implantable mesh 710 including grip members are molded to form a wet foam-3D mesh composite 760, the wet foam layer 720a may be dried into a dry compressible foam 720b by drying the combined wet foam layer 720a and mesh in a 3D configuration 710b in an oven, such as a forced air oven. The wet foam-3D mesh composite 760 can be dried at a temperature between 10 to 100°C, preferably to 20 to 50°C for up to 5 minutes to 16 hours, preferably to 2 to 4 hours to form a dry compressible foam on the mesh forming a composite implant including a non-planar or three-dimensional self-fixating mesh.

Following drying, the one or more securement devices 768, molds 750a, 750b, and inert sheet 733 can be removed from the dry foam-3D mesh composite implant 770, prior to sterilizing. In addition, the dry foam-3D mesh composite implant 770 can be trimmed as needed, e.g., to remove any rough edges formed during manufacturing. The trimming can be performed with any suitable cutting device, such as a scissor, knife, scalpel, laser, etc.

The dry compressible foam surface density on the composite implant may range from about: 0.1mg/cm² to 10mg/cm²; 0.5mg/cm² to 5mg/cm²; or 1mg/cm² to 3mg/cm².

The dry compressible foam may represent from about 5% to 50%; 10% to 40%; or 15% to 30% of the implant mass. Once formed, the composite implants described herein may be sterilized and packaged using an suitable manner known to those skilled in the art.

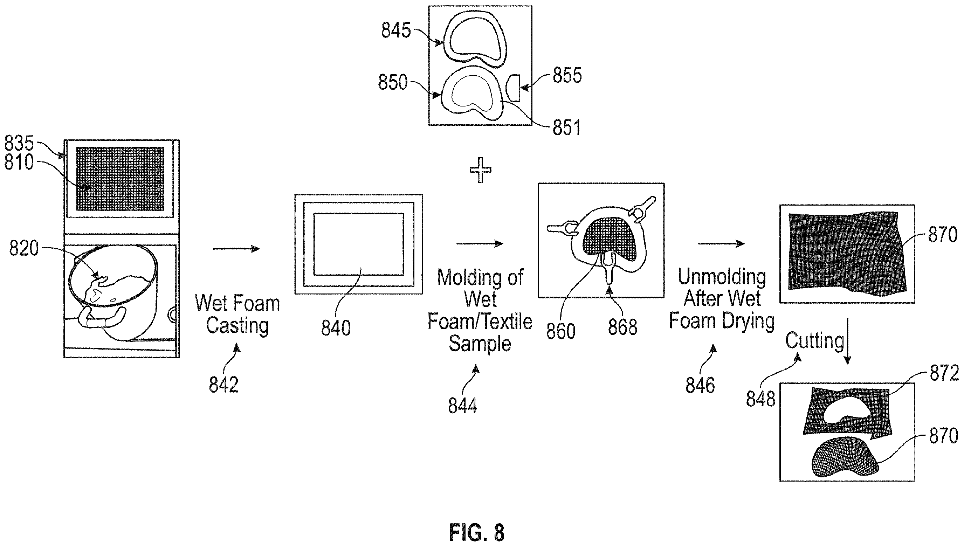

In some embodiments, as shown in FIG. 8, a planar implantable surgical mesh 810 may be combined with a frame assembly 835 before being combined with a wet foam material 820. For example, in some embodiments, at step 840, a planar implantable surgical mesh 810 may be positioned inside a frame cavity of frame 835 and on top of a removable inert base sheet (not shown) prior to being combined with wet foam material 820. In some embodiments, the mesh 810 may be a self-fixating mesh with the grip members facing upward and/or positioned opposite the base sheet.

As further shown in FIG. 8, at step 842, the wet foam material 820 may be applied directly to the planar mesh 810 (and/or grip members) inside the cavity of the frame 835 and leveled across the top of the frame 835 to from a wet foam-planar mesh composite 840. The frame 835 may be removed after the wet foam 820 is cast and/or leveled across the mesh 810. The frame 835 may be removed before molding. The wet foam-planar mesh composite 840 may remain on the base sheet.

Next, at step 844, at least a first mold 850, particularly a solid mold, may be applied to the base sheet side of the wet foam-planar mesh composite 840 to transition at least the planar mesh 810 into a three-dimensional (3D) and/or non-planar configuration thereby forming a wet foam-3D mesh composite 860. In some embodiments, an outer edge 851 of the first mold 850 defines a mold surface area that may be generally equal to or larger than a surface area defined by a perimeter of the wet foam-3D mesh composite 860.

Strapping 845 may be positioned on and/or around an outer edge 851 of the first mold 850 including the wet foam-3D mesh composite 860. The strapping 845 may or may not cover a portion of the wet foam. A close piece 855 is designed to overlap a portion of the strapping 845 along the outer edge 851, particularly along a curved portion of the outer edge 851 of the first mold 850. The close piece 855 may be configured to further stretch the wet foam-3D mesh composite 860 on the first mold 850. One or more clamps 868 may further be added to secure the wet foam-3D mesh composite 860 to any, if not all, of the first mold 850, the strapping 845, and/or the close piece 855. The clamped wet foam-3D mesh composite 860 may then be dried to form a dry foam-3D mesh composite implant 870.

Following drying, as shown at step 846 of Fig. 8, any strapping, close piece, mold(s), sheet(s), and/or clamp(s) may be removed from the dry foam-3D mesh composite 870. In some embodiments, as further shown in Fig. 8 at step 848, the dry-foam-3D mesh composite 870 may further be trimmed and/or cut to remove a portion of the outer edge 872 of the dry foam-3D mesh composite 870.

In the dry format, the layer of dry foam holds the mesh in the non-planar or 3D configuration. Once formed, the dry foam-3D mesh composite implant 870 (composite implant) may be sterilized and/or packaged using any suitable manner known to those skilled in the art.

### Composite Implant

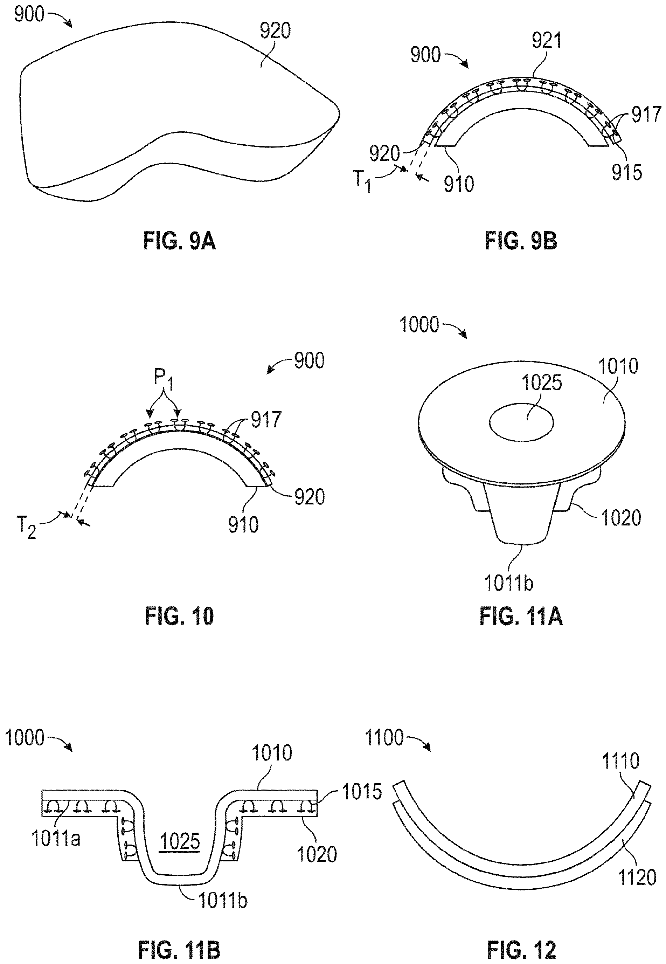

FIGS. 9A-12 depict various non-limiting examples of a dry three-dimensional composite implant as described herein. In FIGS. 9A-9B, the three-dimensional composite implant 900 includes a layer of a three-dimensional self-fixating mesh 910 with a layer of compressible foam 920 attached thereto via grip members 915. In some embodiments, the self-fixating mesh 910 and the compressible foam 920 each generally define a curved, domed, and/or multiplanar contour. In some embodiments, the mesh body of the self-fixating mesh may define an anatomical three-dimensional configuration, such as a Dextile^{®} mesh (Medtronic), further including grip members extending from at least one face thereof.

FIG. 9B depicts a schematic cross-sectional view of a composite surgical implant 900 including at least an implantable mesh 910 and a compressible foam 920. The compressible foam 920 covers at least the head portion 917 of one or more, and particularly a plurality of grip members 915, thereby preventing the grip member(s) 915 from attaching to tissue and/or becoming entangled with other portions of the mesh 910 when overlapped.

In FIG. 9B, the compressible foam 920 is shown in a first initial expanded or noncompressed foam configuration defining a first thickness T₁. In the first configuration, the grip members 915 may be sufficiently buried in the foam 920 such that the grip members 915 are hidden and/or concealed by the foam 920 from at least a top view of the implant 900. In the first configuration, the grip members 915 are in a non-gripping or inactive configuration.

FIG. 10 depicts a schematic cross-sectional view of the composite surgical implant 900 of FIGS. 9A-9B after a force or pressure Pi, as indicated by the arrow in FIG. 10, has been applied to the compressible foam 920. As illustrated, the compressible foam 920 is in a second subsequent and/or compressed configuration defining generally a second subsequent thickness T₂ which is less than first thickness T₁. The dry compressible foam 920 maintains the second configuration even after the force or pressure P₁ is removed (and prior to any dissolution of the foam in vivo).

In some embodiments, as indicated by the arrow in FIG. 10, the force or pressure P₁ may be applied directly to a first outer surface 921 of the foam 920 in a direction generally towards the mesh 910 (and/or the first or second mesh face). Although the arrow depicts the force or pressure P₁ being applied generally perpendicular to the foam 920 (and/or the first outer surface), it is envisioned that in some embodiments the force or pressure P₁ may alternatively be applied at any angle relative to the first outer surface 921 of the foam 920. Some non-limiting examples include the pressure being applied at an angle ranging from 45 to 135 degrees and/or being slid generally parallel to and directly across at least some portion of the first outer surface 921 of the foam 920.

FIG. 10 further depicts that in some embodiments the compressible foam 920 may be compressed evenly across the entire top surface 921, the mesh 910 and/or the grip members 915 in the second configuration. However, in some embodiments, the foam 920 can be transitioned into the second compressed configuration on only limited portions of the top surface 921, the mesh 910 and/or the grip members 915.

As illustrated in FIGS. 9B-10, by "compressible," the foam, in a dried form and upon an application of pressure thereto, is configured to transition from a first foam having a first or initial thickness T₁ to a second foam having a second or subsequent thickness T₂, wherein the second or subsequent thickness is smaller or thinner than the first or initial thickness and the second or subsequent thickness is maintained after the application of pressure is removed. The second or subsequent thickness may also be maintained prior to initial absorption or dissolution of the compressible foam in vivo.

In some embodiments, the compressible foam in the second foam configuration may not be on the second free end, and particularly the head portion, of the one or more grip members thereby directly exposing the grip member to attach to tissue in an active gripping configuration. In the active gripping configuration, the second free end, and particularly the head portion, of the one or more grip members are free of the foam and are configured to attach directly to tissue or other portions of the mesh (or a second mesh or mesh flap).

In some embodiments, the compressible foam in the second foam configuration may still cover the second free end, and particularly the head portion, of the one or more grip members thereby preventing the grip member to attach to tissue in an inactive gripping configuration. However, because there is the foam surrounding the head portion of the grip members in the second configuration is thinner, it may take less time for the foam to dissolve in vivo than in the first configuration, thereby allowing the grip members to attach directly to the tissue or other portions of the mesh (or a second mesh or mesh flap) sooner than in the first configuration.

The compressible foam, in both the first and second configurations, is a porous structure including open and interconnected pores. By "open and interconnected pores," the foam has an open porosity that can be accessed from outside of the foam, and that the pores communicate with one another to form a three-dimensional network throughout the foam.

The compressible foams described herein may be constituted of at least one biocompatible polymeric foaming agent, and optionally at least one biocompatible plasticizer, at least one biocompatible additive, or both. A bioactive agent may also be included.

Each of the foaming agent, the plasticizer, and the additive being soluble within physiological conditions at the site of the soft tissue repair, i.e., the site of a hernia, prolapse, or stoma, or similar fluids such as saline. By "soluble within physiological conditions of the site," the selected material is soluble according to the pH, temperature and/or chemical agents components of biologic fluids on the hernia site. For example, in some embodiments, the dry foam may include gelatins or collagen that are soluble at 37°C but not at 20°C, , and/or the dry foam may include sodium alginates soluble into a fluid containing a high quantity of sodium salts but not into a fluid containing a high quantity of calcium salts.

In some embodiments, the compressible foams described herein may be constituted of at least one biocompatible polymeric foaming agent and at least one biocompatible plasticizer.

In some embodiments, the compressible foams described herein may be constituted of at least one biocompatible polymeric foaming agent and at least one biocompatible additive.

The compressible foams described herein may be constituted of at least one biocompatible polymeric foaming agent, at least one biocompatible plasticizer, and at least one biocompatible additive.

A biocompatible polymeric foaming agent is configured to aid in forming a foam from an aqueous solution as provided in more detail hereinbelow. Some suitable non-limiting examples of foaming agents include proteins such as gelatins, collagens, , poloxamers, polysorbates, methylcellulose (MC), hydroxypropyl methylcellulose (HPMC), hydroxypropyl cellulose (HPC), hydroxyethyl cellulose (HEC) and mixtures thereof.

The addition of at least one biocompatible plasticizer provides flexibility and softness to the dried compressible foam such that compressible foam is soft and pliable. Some non-limiting suitable examples of biocompatible plasticizers include polyhydric alcohols such as glycerol, sorbitol, xylitol, mannitol, propylene glycol, polyethylene glycol, and mixtures thereof. In some embodiments, the compressible foam includes a plasticizer such as glycerol, sorbitol, or both.

The addition of at least one biocompatible additive may provide mechanical strength to the dried compressible foam and/or may serve as thickener (increase viscosity of the solution) during production of the compressible foam. In some embodiments, the additive is a non-polymeric additive. In some embodiments, the additive is a polymeric additive. Some non-limiting suitable examples of biocompatible additives include alginates, pectins, carrageenans, cellulose based derivatives such as cellulose ethers such as methylcellulose (MC), hydroxypropyl methylcellulose (HPMC), hydroxypropyl cellulose (HPC), hydroxyethyl cellulose (HEC), polysaccharides gums such as xanthan gum and guar gum, dextrans, hyaluronans and hyaluronate salts, pullulans, maltodextrins, gelatins, collagens, polyvinyl-alcohol and polyethylene-glycol, and mixtures thereof.

In some embodiments, the wet foam may include a HPMC foaming agent, glycerol plasticizer, and sodium hyaluronates additives.

In some embodiments, the compressible foam includes collagen compounds foaming agent and glycerol plasticizer.

As shown in FIGS. 11A-11B, in some embodiments, the three-dimensional composite implant 1000 may be useful as a surgical plug wherein the implant 1000 includes a three-dimensional self-fixating mesh 1010 defining a generally U-shaped cross-section configured to plug a soft tissue opening. In some embodiments, the composite implant 1000 may include a plurality of grip members 1015 extending from at least a first portion 1011a of the first face 1011 of the mesh 1010, and a dry compressible foam 1020 positioned on the grip members. A second portion 1011b of the first face 1011 free of grip members and/or foam. The composite implant, including particularly the grip members and/or foam, are depicted in an inactive gripping configuration. The surgical plug implant 1000 may further define a channel or cavity 1025.

As shown in FIG. 12, in some embodiments, the 3D composite implant 1100 may include a 3D mesh free of grip members (i.e., non-self-fixating mesh) 1110 and a dry compressible foam 1020. In some embodiments, the dry compressible foam 1020 may be partially embedded into the 3D mesh free of grip members 1110.

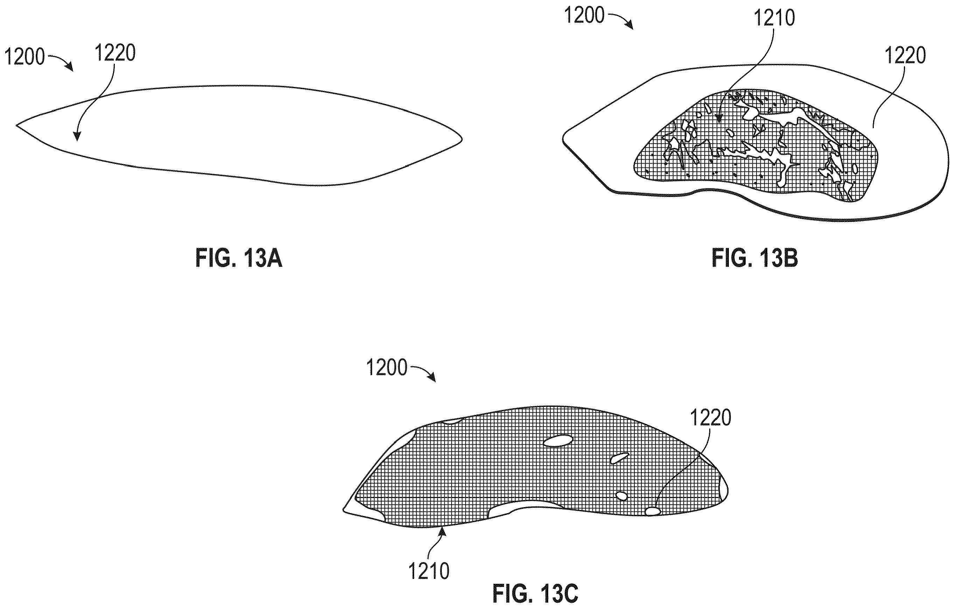

As shown in FIGS. 13A-13C, in some embodiments, a three-dimensional composite implant 1200 as described herein may be configured to transition from a three-dimensional configuration (in the dry foam format) back to a generally planar configuration as the dry foam is hydrated and/or absorbed either prior to or following implantation. Particularly, FIG. 13A depicts a dry 3D composite implant 1200 including a dry 3D compressible foam 1220 on a 3D mesh (with or without grips). As illustrated in FIG. 13B, when at least a portion of the dry 3D composite implant 1220 is hydrated, such as with water, saline, or bodily fluids, the dry foam 1220 may dissolve exposing the 3D mesh 1210. As further illustrated in FIG. 13C, the mesh 1210 may begin to return back to a planar non-3D configuration when a predominant, if not complete, amount of the foam is dissolved/absorbed/hydrated. The methods of forming and/or using the composite implants described herein may further include the step transitioning a three-dimensional composite implant (and/or 3D mesh) into a generally planar mesh as the dry foam is hydrated, dissolved, and/or absorbed.

### Methods of Use

The composite implants described herein are useful for the repair or treatment of soft tissue openings or wounds. Some non-limiting examples include hernia repair, prolapse repair, dural repair, ostomy repair, and the like. In some embodiments, the composite surgical implants described herein are useful for hernia repairs selected from ventral hernias, inguinal hernias, umbilical hernias, etc.

The soft tissue repair or treatment may be performed via open or laparoscopic procedure. In some embodiments, the soft tissue repair or treatment may be for a hernia repair via laparoscopic procedure, such as transabdominal preperitoneal (TAPP) repair or totally extraperitoneal (TEP) repair.

In some embodiments, the composite implants may be used in a method of soft tissue repair. The method may include inserting a composite implant into a site of implantation of a patient near a wound in the soft tissue in need of repair, the composite implant including a three-dimensional (3D) mesh and compressible foam as described herein having a first face and a second face opposite the first face, a plurality of grip members extending from the first face of the mesh, each of the plurality of grip members including a body extending between a first end attached to the mesh and a second end free of the mesh, and a compressible foam covering at least the second ends of the plurality of grip members, the compressible foam configured to transition from a first configuration having a first thickness to a second configuration having a second thickness smaller than the first thickness, and exposing the second ends of the plurality of grip members. Following insertion, the method further includes: orienting the composite three-dimensional implant across the wound in the soft tissue; applying pressure to a portion of the dry compressible foam to transition from the first configuration to the second configuration having a second thickness less than the first thickness thereby exposing the second ends of at least some portion of the plurality grip members; and securing the grip members including the compressible foam in the second configuration to the tissue taking benefit of the presence of body fluids.

The dry compressible foam in both the first and second thicknesses may dissolve in the physiologic fluids found in the tissue. The dry compressible foam in the second compressed configuration may dissolve faster than the compressible foam in the first expanded configuration.

In some embodiments, the dry compressible foam, in at least the second configuration and optionally the first configuration, may be configured to substantially dissolve, i.e., greater than 90% (if not 100%) of the dry compressible foam, in physiologic fluids in less than 30 minutes, and in some embodiments less than 15 minutes. In some embodiments, the dry foam 30 substantially dissolves in physiologic fluids in less than 5 minutes.

Upon implantation, and exposure to physiologic fluids, the dry compressible foam in either configuration will begin to hydrate. Following resorption or dissolution of the compressible foam, from either configuration, the gripping performance of the three-dimensional composite implant is generally equal to the gripping performance of the planar self-fixating mesh prior to being combined with the compressible foam.

Prior to inserting the three-dimensional composite implant, the methods of soft tissue repair may further include forming an incision on the patient prior to inserting the composite implant and optionally positioning a trocar in the incision for inserting the composite implant therethrough. In addition, the method may further include rolling or folding the composite three-dimensional implant prior to inserting into the site of implantation and unrolling or unfolding the composite three-dimensional implant after being inserted into the site of implantation.

In some embodiments, the step of applying pressure to a portion of the compressible foam may include applying the pressure: directly to the first outer surface of the compressible foam; indirectly to the second face of the mesh opposite the first face of the mesh including the compressible foam; or both, in contact with biological tissues.

In some embodiments, the step of applying pressure to a portion of the compressible foam may include applying the pressure directly to outer surface of the compressible foam in contact with biological tissues. The pressure may be applied directly to the foam: at an angle generally perpendicular position relative to a longitudinal axis of the compressible foam; at an angle ranging from about 15° to about 75° relative to a longitudinal axis of the compressible foam; or by sliding the pressure across the first outer surface of the compressible foam.

In some embodiments, the step of applying pressure to a portion of the compressible foam in contact with biological tissues may include applying the pressure indirectly to the second face of the mesh opposite the first face of the mesh including the compressible foam: at an angle generally perpendicular position relative to a longitudinal axis of the mesh (or the compressible foam); at an angle ranging from about 15° to about 75° relative to a longitudinal axis of the mesh (or the compressible foam); or by sliding the pressure across the second face of the mesh.

In some embodiments, the step of applying pressure may further include applying pressure to only a first portion of the compressible foam to transition from the first configuration to the second configuration prior to orienting the composite implant and applying pressure to a second different portion of the compressible foam after orienting the composite implant so the first portion of foam in the second configuration allows the grip members positioned thereon to attach to the tissue to anchor the first portion in place while the rest of the composite implant is adjusted or readjusted for proper orienting.

Once the composite three-dimensional implant is properly oriented and the grip members including the compressible foam in the second configuration are attached to the surrounding tissue, the method of repair may further include: fastening the implantable mesh to tissue around the wound with suitable surgical fasteners, such as sutures, staples, adhesives, tacks, and the like; closing the incision, or both.

The implants described herein are designed to be used in an open or laparoscopic surgical procedure. The surgical procedure may be performed directly by a person, e.g., surgeon or other medical staff member, or via the assistance of a surgical robot. Non-limiting examples of the at least one surgical robot include: single and multi-port robots manufactured by Intuitive Surgical^{™} (e.g., the DaVinci^{™}); and the Hugo^{™} from Medtronic^{™}.

In some embodiments, the implants described herein may be designed to treat and/or repair soft tissue defects, such as hernias or prolapses. In some embodiments, the implants may be designed to treat and/or repair hernias in at least one of the intraperitoneal, preperitoneal, retromuscular spaces. The implants described herein may be one or more of an onlay implant, an inlay implant, an underlay implant, a retromuscular implant, a preperitoneal implant, or an intraperitoneal implant. The implants described herein may be used in one or more hernia-related procedures including trans-abdominal preperitoneal procedures (TAPP), totally extraperitoneal procedures (TEP), trans-inguinal preperitoneal procedures (TIPP), minimal open preperitoneal procedures (MOPP), intraperitoneal onlay mesh procedures (IPOM), laparoscopic intracorporeal rectus aponeuroplasty procedures (LIRA), subcutaneous onlay laparoscopic approach procedures (SCOLA), transabdominal retromuscular umbilical prosthetic procedures (TARUP), and the like. Each of these procedures may be performed directly by a surgeon or medical staff member and/or via surgical robotic systems/devices. For example, TAPP may include robotic trans-abdominal preperitoneal procedures (rTAPP) and/or TEP may include extended/enhanced view totally extraperitoneal procedures (eTEP), etc.

The implants described herein are designed to be easily rolled and/or folded into a compact configuration suitable for passing through a trocar or cannula of various sizes. For example, the implants, in a compacted or non-compacted configuration may be designed to pass through a trocar or cannula having a diameter ranging from about 5mm to about 15mm. In some embodiments, the diameter of the trocar or cannula may be 8mm, 10mm, 11mm, or 12mm. The trocar or cannula may be reusable or disposable. The trocar may be made of any biocompatible material including metals, such as stainless steel and/or any suitable biocompatible polymeric material.

It will be understood that various modifications may be made to the embodiments of the presently disclosed composite implants. For example, embodiments described herein directed to self-fixating mesh may be intended to also encompass non-self-fixating mesh and vice versa. Therefore, the above description should not be construed as limiting, but merely as exemplifications of embodiments. Those skilled in the art will envision other modifications within the scope and spirit of the present disclosure.

### EXAMPLE 1

An implantable planar surgical mesh, having a size of 24cm × 18cm including a plurality of grip members extending from the first face of the mesh as described in WO01/81667 was provided. The grips members being produced from a monofilament yarn of bioresorbable material such as polylactic acid (PLA). The implantable surgical mesh was cleaned with water to remove the residual oil at the surface of yarns. A foam layer, in the form of dry compressible foam, was combined with the grip members of the implantable mesh to form a composite implant, in the following manner:

### 1) Preparation of the aqueous solution of soluble materials within physiological conditions in order to form a wet foam:

An aqueous solution containing 2.5 weight percent of hydroxypropyl methylcellulose, 2.0 weight percent sodium hyaluronate and 2.0 weight percent of glycerol was prepared as follows.

First, the hydroxypropyl methylcellulose was dispersed in heated sterile water (80°C) to form an HPMC aqueous dispersion. Glycerol was added to the HPMC aqueous dispersion and cooled at room temperature for 2 hours. Then, the sodium hyaluronate was added to the HPMC-Glycerol solution and the solution was mixed for 16 hours at room temperature. The hydroxypropyl methylcellulose used was commercially available from The ShinEtsu Company under the trademark PHARMACOAT^{®} 603. The glycerol used was commercially available from The Merck Company under the Trademark EMPROVE^{®} exp anhydrous glycerol. The sodium hyaluronate used was provided by the HTL Biotechnology company with an intrinsic viscosity of 1.02m³/Kg.

### 2) Preparation of a wet foam material

The obtained aqueous solution in the preceding steps was poured into a mixer bowl (Kitchen machine Cooking Chef from Kenwood company). The solution was whisked for 2 minutes at room temperature to introduce air bubbles into the aqueous solution. Following the incorporation of air bubbles, a wet foam material was formed. The wet foam density of the wet foam material was about 0.26g/cm³.

### 3) Combining the wet foam material with grip members of the planar mesh using a frame assembly

A stainless-steel rectangular frame (24cm × 18cm externally; 20xm × 14cm internally dimensions) having a thickness of 1.5mm was placed on top of a rectangular silicon sheet (24cm × 18cm) having a thickness of 0.51mm. The silicon sheet was provided by the Rogers corporation company. Then, the wet foam was cast into the frame assembly and was leveled down across the top of the frame using a metallic bar to provide a consistent foam thickness. The metallic frame was removed from the wet foam layer and the silicon sheet. The planar implantable mesh was laid down over the top outer surface of the wet foam layer with the grip members facing downward towards the wet foam. The mesh was rolled to embed the grip members in the wet foam forming a wet foam-planar mesh composite.

### 4) Mold the wet foam-planar mesh composite

The assembly constituted of the silicon sheet and the wet foam-planar mesh composite was put onto a solid mold in polyethylene terephthalate (PET) having dimensions corresponding to the product Dextile^{™} Anatomical Mesh (Large size, right shape). A second porous honeycomb mold in Polyether ether ketone (PEEK) was placed against the mesh with the composite positioned between the two molds and the two molds were pressed towards each other to form a wet foam-3D mesh composite. The two molds were clamped to the composite via two (2) metallic clamps to keep the 3D shape during the drying of the wet foam.

### 5) Drying the wet foam-3D mesh composites

The two molds assembled together and containing the implantable mesh in a three-dimensional configuration and the wet foam material were then placed into an oven (28°C/35% relative humidity) with the honeycomb part exposed to the air flow and dried 16 hours to form a dry foam-3D mesh composite implant.

### 6) Cutting of the mesh dry foam-3D mesh composite implant after drying

After drying, the metallic clips, the two molds, and the silicon sheet were removed from the composite implant. Then, the edges of the implant were cut with scissors to have a mesh body defining a three-dimensional contour identical to a Dextile^{™} Anatomical Mesh (Large size, right shape), the mesh body further including bioabsorbable grip members, i.e., PLA grip members, covered with a layer of a dry compressible foam.

### 7) Sterilization of the composite implant

The three-dimensional composite implants were packaged into clamshells designed to the dimensions of the 3D implant and sterilized using EtO at 38°C for 5 hours, Delta P=400mBar(clam shell were put into Tyvek^{®} bags for the sterilization). After sterilization, the Tyvek^{®} bags were sealed in foil pouches and kept at room temperature until used.

### EXAMPLE 2

An implantable planar surgical mesh, having a size of 24cm × 18cm including a plurality of grip members extending from the first face of the mesh as described in WO01/81667 was provided. The grips members being produced from a monofilament yarn of bioresorbable material such as polylactic acid (PLA). The implantable surgical mesh was cleaned with water to remove the residual oil at the surface of yarns. A foam layer, in the form of dry compressible foam, was combined with the grip members of the implantable mesh to form a composite implant, in the following manner:

### 1) Preparation of the aqueous solution of soluble materials within physiological conditions in order to form a wet foam:

An aqueous solution containing 2.5 weight percent of collagen compound, and 0.25 weight percent of glycerol was prepared as follows.

First, the collagen and the glycerol were poured in heated sterile water (33°C). The solution was mixed for 4 hours at 33°C. The collagen used is Type I porcine collagen extracted from pork skin by solubilization at acid pH or by pepsin digestion and purified by saline precipitation according to known techniques (Sofradim Production). The glycerol used was commercially available from The Merck Company under the Trademark EMPROVE^{®} exp anhydrous glycerol.

### 2) Preparation of a wet foam material

The obtained aqueous solution in the preceding steps was poured into a mixer bowl (Kitchen machine Cooking Chef from Kenwood company). The solution was whisked for 2 minutes at room temperature to introduce air bubbles into the aqueous solution. Following the incorporation of air bubbles, a wet foam material was formed. The wet foam density of the wet foam material was about 0.20g/cm³.

### 3) Combining the wet foam material with grip members of the planar mesh using a frame assembly

A stainless-steel rectangular frame (24cm × 18cm externally; 20xm × 14cm internally dimensions) having a thickness of 1.0mm was placed on top of a rectangular silicon sheet (24cm × 18cm) having a thickness of 0.51mm. The silicon sheet was provided by the Rogers corporation company. Then, the wet foam was cast into the frame assembly and was leveled down across the top of the frame using a metallic bar to provide a consistent foam thickness. The metallic frame was removed from the wet foam layer and the silicon sheet. The planar implantable mesh was laid down over the top outer surface of the wet foam layer with the grip members facing downward towards the wet foam. The mesh was rolled to embed the grip members in the wet foam forming a wet foam-planar mesh composite.

### 4) Mold the wet foam-planar mesh composite

The assembly constituted of the silicon sheet and the wet foam-planar mesh composite was put onto a solid mold in polyethylene terephthalate (PET) having dimensions corresponding to the product Dextile^{™} Anatomical Mesh (Large size, right shape). A second porous honeycomb mold in Polyether ether ketone (PEEK) was placed against the mesh with the composite positioned between the two molds and the two molds were pressed towards each other to form a wet foam-3D mesh composite. The two molds were clamped to the composite via two (2) metallic clamps to keep the 3D shape during the drying of the wet foam.

### 5) Drying the wet foam-3D mesh composites

The two molds assembled together and containing the implantable mesh in a three-dimensional configuration and the wet foam material were then placed into an oven (20°C/40% relative humidity) with the honeycomb part exposed to the air flow and dried 16 hours to form a dry foam-3D mesh composite implant.

### 6) Cutting of the mesh dry foam-3D mesh composite implant after drying

After drying, the metallic clips, the two molds, and the silicon sheet were removed from the composite implant. Then, the edges of the implant were cut with scissors to have a mesh body defining a three-dimensional contour identical to a Dextile^{™} Anatomical Mesh (Large size, right shape), the mesh body further including bioabsorbable grip members, i.e., PLA grip members, covered with a layer of a dry compressible foam.

### 7) Sterilization of the composite implant

The three-dimensional composite implants were packaged into clamshells designed to the dimensions of the 3D implant and sterilized using EtO at 38°C for 5 hours, Delta P=400mBar (clam shell were put into Tyvek^{®} bags for the sterilization). After sterilization, the Tyvek^{®} bags were sealed in foil pouches and kept at room temperature until used.

### EXAMPLE 3

An implantable planar surgical mesh, having a size of 24cm × 18cm including a plurality of grip members extending from the first face of the mesh as described in WO01/81667 was provided. The grips members being produced from a monofilament yarn of bioresorbable material such as polylactic acid (PLA). The implantable surgical mesh was cleaned with water to remove the residual oil at the surface of yarns. A foam layer, in the form of dry compressible foam, was combined with the grip members of the implantable mesh to form a composite implant, in the following manner:

### 1) Preparation of the aqueous solution of soluble materials within physiological conditions in order to form a wet foam:

An aqueous solution containing 6.0 weight percent of collagen compound, and 1.5 weight percent of glycerol was prepared as follows.

First, the collagen and the glycerol were poured in heated sterile water (40°C). The solution was mixed for 1 hour at 40°C. The collagen used is Type I porcine collagen extracted from pork skin by solubilization at acid pH or by pepsin digestion and purified by saline precipitation according to known techniques (Sofradim Production). The glycerol used was commercially available from The Merck Company under the Trademark EMPROVE^{®} exp anhydrous glycerol.

### 2) Preparation of a wet foam material

The obtained aqueous solution in the preceding steps was poured into a mixer bowl (Kitchen machine Cooking Chef from Kenwood company). The solution was whisked for 5 minutes at room temperature to introduce air bubbles into the aqueous solution. Following the incorporation of air bubbles, a wet foam material was formed. The wet foam density of the wet foam material was about 0. 10g/cm³.

### 3) Combining the wet foam material with grip members of the planar mesh using a frame assembly

A stainless-steel rectangular frame (24cm × 18cm externally; 20xm × 14cm internally dimensions) having a thickness of 2.0mm was placed on top of a rectangular silicon sheet (24cm × 18cm) having a thickness of 0.51mm. The silicon sheet was provided by the Rogers corporation company. Then, the wet foam was cast into the frame assembly and was leveled down across the top of the frame using a metallic bar to provide a consistent foam thickness. The metallic frame was removed from the wet foam layer and the silicon sheet. The planar implantable mesh was laid down over the top outer surface of the wet foam layer with the grip members facing downward towards the wet foam. The mesh was rolled to embed the grip members in the wet foam forming a wet foam-planar mesh composite.

### 4) Mold the wet foam-planar mesh composite

The assembly constituted of the silicon sheet and the wet foam-planar mesh composite was put onto a solid mold in polyethylene terephthalate (PET) having dimensions corresponding to the product Dextile^{™} Anatomical Mesh (Large size, right shape). A second porous honeycomb mold in Polyether ether ketone (PEEK) was placed against the mesh with the composite positioned between the two molds and the two molds were pressed towards each other to form a wet foam-3D mesh composite. The two molds were clamped to the composite via two (2) metallic clamps to keep the 3D shape during the drying of the wet foam.

### 5) Drying the wet foam-3D mesh composites

The two molds assembled together and containing the implantable mesh in a three-dimensional configuration and the wet foam material were then placed into an oven (20°C/40% relative humidity) with the honeycomb part exposed to the air flow and dried 16 hours to form a dry foam-3D mesh composite implant.

### 6) Cutting of the mesh dry foam-3D mesh composite implant after drying

After drying, the metallic clips, the two molds, and the silicon sheet were removed from the composite implant. Then, the edges of the implant were cut with scissors to have a mesh body defining a three-dimensional contour identical to a Dextile^{™} Anatomical Mesh (Large size, right shape), the mesh body further including bioabsorbable grip members, i.e., PLA grip members, covered with a layer of a dry compressible foam.

### 7) Sterilization of the composite implant

The three-dimensional composite implants were packaged into clamshells designed to the dimensions of the 3D implant and sterilized using EtO at 38°C for 5 hours, Delta P=400mBar (clam shell were put into Tyvek^{®} bags for the sterilization). After sterilization, the Tyvek^{®} bags were sealed in foil pouches and kept at room temperature until used.

### EXAMPLE 4

An implantable planar surgical mesh, having a size of 24cm × 18cm including a plurality of grip members extending from the first face of the mesh as described in WO01/81667 was provided. The grips members being produced from a monofilament yarn of bioresorbable material such as polylactic acid (PLA). The implantable surgical mesh was cleaned with water to remove the residual oil at the surface of yarns. A foam layer, in the form of dry compressible foam, was combined with the grip members of the implantable mesh to form a composite implant, in the following manner:

1) Preparation of the aqueous solution of soluble materials within physiological conditions in order to form a wet foam:

An aqueous solution containing 6.0 weight percent of collagen compound, and 1.5 weight percent of glycerol was prepared as follows.

First, the collagen and the glycerol were poured in heated sterile water (40°C). The solution was mixed for 1 hour at 40°C. The collagen used is Type I porcine collagen extracted from pork skin by solubilization at acid pH or by pepsin digestion and purified by saline precipitation according to known techniques (Sofradim Production). The glycerol used was commercially available from The Merck Company under the Trademark EMPROVE^{®} exp anhydrous glycerol.

### 2) Preparation of a wet foam material

The obtained aqueous solution in the preceding steps was poured into a mixer bowl (Kitchen machine Cooking Chef from Kenwood company). The solution was whisked for 5 minutes at room temperature to introduce air bubbles into the aqueous solution. Following the incorporation of air bubbles, a wet foam material was formed. The wet foam density of the wet foam material was about 0.10g/cm³.

### 3) Combining the wet foam material with grip members of the planar mesh using a frame assembly

The planar implantable mesh was placed with the grips upward on top of a rectangular silicon sheet (24cm × 18cm) having a thickness of 0.51mm. The silicon sheet was provided by the Rogers corporation company. A stainless-steel rectangular frame (24cm × 18cm externally; 20xm × 14cm internally dimensions) having a thickness of 2.0mm was placed on top of the planar implantable mesh. Then, the wet foam was cast into the frame assembly and was leveled down across the top of the frame using a metallic bar to provide a consistent foam thickness to form a wet foam-planar mesh composite. The metallic frame was removed from the wet foam-planar mesh composite and the silicon sheet.

### 4) Mold the wet foam-planar mesh composite

The assembly constituted of the silicon sheet and the wet foam-planar mesh composite was put onto a solid mold in acrylonitrile butadiene styrene (ABS) with a central part having dimensions corresponding to the product Dextile^{™} Anatomical Mesh (Large size, right shape) and a flat edge around the central part (2.5cm width). Then, a strapping (2.25cm width) in acrylonitrile butadiene styrene (ABS) was placed on top of the wet-foam planar mesh composite part covering the flat edge of the solid mold. A piece to close the strapping in acrylonitrile butadiene styrene (ABS) (8cm X 3cm) was inserted through the strapping near the central curve of the Dextile^{™} Anatomical Mesh solid mold to increase the stretching of the wet-foam planar mesh composite. The solid mold and the strapping were clamped to the composite via two 3 metallic clamps to maintain the stretching of the wet-foam planar mesh composite during the drying of the wet foam.

### 5) Drying the wet foam-3D mesh composites

The solid mold, the strapping, and the piece to close the strapping assembled together and containing the implantable mesh in a three-dimensional configuration and the wet foam material were then placed into an oven (20°C/40% relative humidity) with the wet foam part exposed to the air flow and dried 16 hours to form a dry foam-3D mesh composite implant.

### 6) Cutting of the mesh dry foam-3D mesh composite implant after drying

After drying, the metallic clips, the solid mold, the strapping, the piece to close the strapping and the silicon sheet were removed from the composite implant. Then, the edges of the implant were cut with scissors to have a mesh body defining a three-dimensional contour identical to a Dextile^{™} Anatomical Mesh (Large size, right shape), the mesh body further including bioabsorbable grip members, i.e., PLA grip members, covered with a layer of a dry compressible foam.

### 7) Sterilization of the composite implant

The three-dimensional composite implants were packaged into clamshells designed to the dimensions of the 3D implant and sterilized using EtO at 38°C for 5 hours, Delta P=400mBar (clam shell were put into Tyvek^{®} bags for the sterilization). After sterilization, the Tyvek^{®} bags were sealed in foil pouches and kept at room temperature until used.

### EXAMPLE 5

A Parietene^{™} Macroporous Mesh (non-self-fixating) having a size of 20cm × 20cm intended for the repair of hernias or other fascial deficiencies that require the addition of a reinforcing material was provided. A foam layer, in the form of dry compressible foam, was combined with the Parietene^{™} Macroporous Mesh to form a composite implant, in the following manner:

### 1) Preparation of the aqueous solution of soluble materials within physiological conditions in order to form a wet foam:

An aqueous solution containing 2.5 weight percent of collagen compound, and 0.25 weight percent of glycerol was prepared as follows.

First, the collagen and the glycerol were poured in heated sterile water (33°C). The solution was mixed for 4 hours at 33°C. The collagen used is Type I porcine collagen extracted from pork skin by solubilization at acid pH or by pepsin digestion and purified by saline precipitation according to known techniques (Sofradim Production). The glycerol used was commercially available from The Merck Company under the Trademark EMPROVE^{®} exp anhydrous glycerol.

### 2) Preparation of a wet foam material

The obtained aqueous solution in the preceding steps was poured into a mixer bowl (Kitchen machine Cooking Chef from Kenwood company). The solution was whisked for 2 minutes at room temperature to introduce air bubbles into the aqueous solution. Following the incorporation of air bubbles, a wet foam material was formed. The wet foam density of the wet foam material was about 0.20g/cm³.

### 3) Combining the wet foam material with the planar mesh using a frame assembly

A stainless-steel rectangular frame (24cm X 18cm externally; 20xm X 14cm internally dimensions) having a thickness of 1.0mm was placed on top of a rectangular silicon sheet (24cm × 18cm) having a thickness of 0.51mm. The silicon sheet was provided by the Rogers corporation company. Then, the wet foam was cast into the frame assembly and was leveled down across the top of the frame using a metallic bar to provide a consistent foam thickness. The metallic frame was removed from the wet foam layer and the silicon sheet. Parietene^{™} Macroporous Mesh was laid down over the top outer surface of the wet foam layer. The mesh was rolled to embed the yarns (e.g., filaments) of the mesh in the wet foam forming a wet foam-planar mesh composite.

### 4) Mold the wet foam-planar mesh composite

The assembly constituted of the silicon sheet and the wet foam-planar mesh composite was put onto a solid mold in acrylonitrile butadiene styrene (ABS) having dimensions corresponding to the product Dextile^{™} Anatomical Mesh (Large size, right shape). A second porous honeycomb mold in acrylonitrile butadiene styrene (ABS) was placed against the mesh with the composite positioned between the two molds and the two molds were pressed towards each other to form a wet foam-3D mesh composite. The two molds were clamped to the composite via two 2 metallic clamps to keep the 3D shape during the drying of the wet foam.

### 5) Drying the wet foam-3D mesh composites

The two molds assembled together and containing the mesh in a three-dimensional configuration and the wet foam material were then placed into an oven (20°C/40% relative humidity) with the honeycomb part exposed to the air flow and dried 16 hours to form a dry foam-3D mesh composite implant.

### 6) Cutting of the mesh dry foam-3D mesh composite implant after drying

After drying, the metallic clips, the two molds, and the silicon sheet were removed from the composite implant. Then, the edges of the implant were cut with scissors to have a mesh body defining a three-dimensional contour identical to a Dextile^{™} Anatomical Mesh (Large size, right shape), the mesh body further covered with a layer of a dry compressible foam.

### 7) Sterilization of the composite implant

The three-dimensional composite meshes were packaged into clamshells designed to the dimensions of the 3D implant and sterilized using EtO at 38°C for 5 hours, Delta P=400mBar (clam shell were put into Tyvek^{®} bags for the sterilization). After sterilization, the Tyvek^{®} bags were sealed in foil pouches and kept at room temperature until used.

### EXAMPLE 6

An implantable planar surgical mesh, having a size of 24cm X 18cm including a plurality of grip members extending from the first face of the mesh as described in WO01/81667 was provided. The grips members being produced from a monofilament yarn of bioresorbable material such as polylactic acid (PLA). The implantable surgical mesh was cleaned with water to remove the residual oil at the surface of yarns. A foam layer, in the form of dry compressible foam, was combined with the grip members of the implantable mesh to form a composite implant, in the following manner:

### 1) Preparation of the aqueous solution of soluble materials within physiological conditions in order to form a wet foam:

An aqueous solution containing 1.0 weight percent of hydroxypropyl methylcellulose, 3.0 weight percent sodium hyaluronate at low intrinsic viscosity, 0.6 weight percent sodium hyaluronate at high intrinsic viscosity and 2.5 weight percent of glycerol was prepared as follows.

First, the hydroxypropyl methylcellulose was dispersed in heated sterile water (80°C) to form an HPMC aqueous dispersion. Glycerol was added to the HPMC aqueous dispersion and cooled at room temperature for 2 hours. Then, the sodium hyaluronates were added to the HPMC-Glycerol solution and the solution was mixed for 16 hours at room temperature. The hydroxypropyl methylcellulose used was commercially available from The ShinEtsu Company under the trademark PHARMACOAT^{®} 603. The glycerol used was commercially available from The Merck Company under the Trademark EMPROVE^{®} exp anhydrous glycerol. The sodium hyaluronates used were provided by the HTL Biotechnology company with intrinsic viscosities of 0.38m³/Kg and 2.74 m3/Kg.

### 2) Preparation of a wet foam material

The obtained aqueous solution in the preceding steps was poured into a mixer bowl (Kitchen machine Cooking Chef from Kenwood company). The solution was whisked for 2 minutes at room temperature to introduce air bubbles into the aqueous solution. Following the incorporation of air bubbles, a wet foam material was formed. The wet foam density of the wet foam material was about 0.25g/cm³.

### 3) Combining the wet foam material with grip members of the planar mesh using a frame assembly

The planar implantable mesh was placed with the grips upward on top of a rectangular silicon sheet (24cm X 18cm) having a thickness of 0.51mm. The silicon sheet was provided by the Rogers corporation company. A stainless-steel rectangular frame (24cm X 18cm externally; 20xm × 14cm internally dimensions) having a thickness of 1.5mm was placed on top of the planar implantable mesh. Then, the wet foam was cast into the frame assembly and was leveled down across the top of the frame using a metallic bar to provide a consistent foam thickness to form a wet foam-planar mesh composite. The metallic frame was removed from the wet foam-planar mesh composite and the silicon sheet.

### 4) Mold the wet foam-planar mesh composite

The assembly constituted of the silicon sheet and the wet foam-planar mesh composite was put onto a solid mold in acrylonitrile butadiene styrene (ABS) with a central part having dimensions corresponding to the product Dextile^{™} Anatomical Mesh (Large size, right shape) and a flat edge around the central part (2.5cm width). Then, a strapping (2.25cm width) in acrylonitrile butadiene styrene (ABS) was placed on top of the wet-foam planar mesh composite part covering the flat edge of the solid mold. A piece to close the strapping in acrylonitrile butadiene styrene (ABS) (8cm X 3cm) was inserted through the strapping near the central curve of the Dextile^{™} Anatomical Mesh solid mold to increase the stretching of the wet-foam planar mesh composite. The solid mold and the strapping were clamped to the composite via two 3 metallic clamps to maintain the stretching of the wet-foam planar mesh composite during the drying of the wet foam.

### 5) Drying the wet foam-3D mesh composites

The solid mold, the strapping, and the piece to close the strapping assembled together and containing the implantable mesh in a three-dimensional configuration and the wet foam material were then placed into an oven (50°C/20% relative humidity) with the wet foam part exposed to the air flow and dried 2 hours to form a dry foam-3D mesh composite implant.

### 6) Cutting of the mesh dry foam-3D mesh composite implant after drying

After drying, the metallic clips, the solid mold, the strapping, the piece to close the strapping and the silicon sheet were removed from the composite implant. Then, the edges of the implant were cut with scissors to have a mesh body defining a three-dimensional contour identical to a Dextile^{™} Anatomical Mesh (Large size, right shape), the mesh body further including bioabsorbable grip members, i.e., PLA grip members, covered with a layer of a dry compressible foam.

### 7) Sterilization of the composite implant

The three-dimensional composite implants were packaged into clamshells designed to the dimensions of the 3D implant and sterilized using EtO at 38°C for 5 hours, Delta P=400mBar (clam shell were put into Tyvek^{®} bags for the sterilization). After sterilization, the Tyvek^{®} bags were sealed in foil pouches and kept at room temperature until used.

### EXAMPLE 7

A Versatex^{™} monofilament mesh having a size of 20cm X 20cm and intended for reinforcement of abdominal wall soft tissue where a weakness exists, in procedures involving abdominal wall hernia repairs was provided. A foam layer, in the form of dry compressible foam, was combined with the Versatex^{™} monofilament mesh to form a composite implant, in the following manner:

### 1) Preparation of the aqueous solution of soluble materials within physiological conditions in order to form a wet foam:

An aqueous solution containing 1.0 weight percent of hydroxypropyl methylcellulose, 2.0 weight percent sodium hyaluronate and 1.0 weight percent of glycerol was prepared as follows.

First, the hydroxypropyl methylcellulose was dispersed in heated sterile water (80°C) to form an HPMC aqueous dispersion. Glycerol was added to the HPMC aqueous dispersion and cooled at room temperature for 2 hours. Then, the sodium hyaluronate was added to the HPMC-Glycerol solution and the solution was mixed for 16 hours at room temperature. The hydroxypropyl methylcellulose used was commercially available from The ShinEtsu Company under the trademark PHARMACOAT^{®} 603. The glycerol used was commercially available from The Merck Company under the Trademark EMPROVE^{®} exp anhydrous glycerol. The sodium hyaluronates used was provided by the HTL Biotechnology company with intrinsic viscosities of 1.60m3/Kg.

### 2) Preparation of a wet foam material

The obtained aqueous solution in the preceding steps was poured into a mixer bowl (Kitchen machine Cooking Chef from Kenwood company). The solution was whisked for 2 minutes at room temperature to introduce air bubbles into the aqueous solution. Following the incorporation of air bubbles, a wet foam material was formed. The wet foam density of the wet foam material was about 0.25g/cm³.

### 3) Combining the wet foam material with the planar mesh using a frame assembly

The Versatex^{™} monofilament mesh was placed on top of a rectangular silicon sheet (24cm X 18cm) having a thickness of 0.51mm. The silicon sheet was provided by the Rogers corporation company. A stainless-steel rectangular frame (24cm × 18cm externally; 20xm × 14cm internally dimensions) having a thickness of 1.5mm was placed on top of Versatex^{™} monofilament mesh. Then, the wet foam was cast into the frame assembly and was leveled down across the top of the frame using a metallic bar to provide a consistent foam thickness to form a wet foam-planar mesh composite. The metallic frame was removed from the wet foam-planar mesh composite and the silicon sheet.

### 4) Mold the wet foam-planar mesh composite

The assembly constituted of the silicon sheet and the wet foam-planar mesh composite was put onto a solid mold in acrylonitrile butadiene styrene (ABS) with a central part having dimensions corresponding to the product Dextile^{™} Anatomical Mesh (Large size, right shape) and a flat edge around the central part (2.5cm width). Then, a strapping (2.25cm width) in acrylonitrile butadiene styrene (ABS) was placed on top of the wet-foam planar mesh composite part covering the flat edge of the solid mold. A piece to close the strapping in acrylonitrile butadiene styrene (ABS) (8cm × 3cm) was inserted through the strapping near the central curve of the Dextile^{™} Anatomical Mesh solid mold to increase the stretching of the wet-foam planar mesh composite. The solid mold and the strapping were clamped to the composite via two 3 metallic clamps to maintain the stretching of the wet-foam planar mesh composite during the drying of the wet foam.

### 5) Drying the wet foam-3D mesh composites

The solid mold, the strapping, and the piece to close the strapping assembled together and containing the implantable mesh in a three-dimensional configuration and the wet foam material were then placed into an oven (50°C/20% relative humidity) with the wet foam part exposed to the air flow and dried 2 hours to form a dry foam-3D mesh composite implant.

### 6)Cutting of the mesh dry foam-3D mesh composite implant after drying

After drying, the metallic clips, the solid mold, the strapping, the piece to close the strapping and the silicon sheet were removed from the composite implant. Then, the edges of the implant were cut with scissors to have a mesh body defining a three-dimensional contour identical to a Dextile^{™} Anatomical Mesh (Large size, right shape), the mesh body covered with a layer of a dry compressible foam.

### 7) Sterilization of the composite implant

The three-dimensional composite implants were packaged into clamshells designed to the dimensions of the 3D implant and sterilized using EtO at 38°C for 5 hours, Delta P=400mBar (clam shell were put into Tyvek^{®} bags for the sterilization). After sterilization, the Tyvek^{®} bags were sealed in foil pouches and kept at room temperature until used.

## Claims

1. A method of manufacturing a three-dimensional composite implant comprising:
combining a wet foam material with a generally planar implantable surgical mesh forming a wet foam-planar mesh composite;
applying a first mold to a first side of the wet foam-planar mesh composite to transition the generally planar implantable surgical mesh into a three-dimensional implantable surgical mesh forming a wet foam-3D mesh composite;
drying the wet foam-3D mesh composite forming a dry foam-3D mesh composite implant; and
removing the mold from the dry foam-3D mesh composite implant.

2. The method of claim 1, wherein combining the wet foam material with the generally planar implantable surgical mesh comprises applying the wet foam material directly to a plurality of filaments along a first face of the generally planar implantable surgical mesh to form a layer of wet foam material thereon.

3. The method of claim 1, wherein applying the first mold to the first side of the wet foam-planar mesh composite further comprises applying a second mold to a second opposite side of the wet foam-planar mesh composite.

4. The method of claim 3, wherein the first mold is a solid mold and the second mold is a porous mold.

5. The method of claim 1, further comprising adding a strapping and a close piece along an outer edge of the first mold, securing the first mold to the wet foam-3D mesh composite with one or more clamps, or both.

6. The method of claim 5, further comprising removing at least one of the first mold, the strapping, the close piece, or the one or more clamps, after drying.

7. The method of claim 1, wherein drying the wet foam-3D mesh composite includes drying the wet foam material and the three-dimensional implantable surgical mesh in an oven.

8. The method of claim 1, wherein the dry foam of the three-dimensional composite implant includes a dry compressible foam configured to transition from a first foam configuration having a first thickness to a second foam configuration having a second thickness smaller than the first thickness, without changing a non-planar configuration of the three-dimensional implantable surgical mesh.

9. The method of claim 1, wherein the generally planar implantable surgical mesh is a generally planar self-fixating mesh including a plurality of grip members extending from the first face thereof.

10. The method of claim 9, further comprising:
combining a frame assembly with the wet foam material prior to combining the wet foam material with the generally planar self-fixating mesh, wherein the frame assembly includes a frame defining a frame cavity and a first sheet of inert material; and,
removing the frame prior to combining the wet foam material and the generally planar self-fixating mesh.

11. The method of claim 10, wherein combining the frame assembly with the wet foam material includes casting, and optionally leveling, a layer of wet foam material inside the frame cavity and on top of the first sheet and combining the wet foam material with the generally planar self-fixating mesh includes applying the plurality of grip members positioned on the first face of the generally planar self-fixating mesh into the layer of wet foam material.

12. The method of claim 11, wherein applying the plurality of grip members of the generally planar self-fixating mesh into the layer of wet foam material includes embedding the plurality of grip members into the wet foam by rolling a second face of the generally planar self-fixating mesh opposite the first face with a roller.

13. The method of claim 9, further comprising combining a frame assembly with the generally planar self-fixating mesh prior to combining the wet foam material with the generally planar self-fixating mesh, wherein the frame assembly includes a frame defining a frame cavity and a first sheet of inert material; and removing the frame prior to applying the first mold to the wet foam-planar mesh composite.

14. The method of claim 13, wherein combining the frame assembly with the generally planar self-fixating mesh includes positioning at least a portion of the generally planar self-fixating mesh on the first sheet and within the frame cavity of the frame prior to combining the wet foam material with the generally planar self-fixating mesh, and combining the wet foam material with the generally planar self-fixating mesh includes casting a layer of foam material on the plurality of grip members positioned on the first face of the generally planar self-fixating mesh.

15. The method of claim 1, further comprising transitioning the three-dimensional implantable surgical mesh into the generally planar implantable surgical mesh as the dry foam is hydrated or absorbed.

16. A three-dimensional composite implant comprising:
a three-dimensional implantable surgical mesh including a first face and a second opposite face, and
a three-dimensional dry foam attached to a plurality of filaments along the first face of the three-dimensional implantable surgical mesh.

17. The three-dimensional composite implant of claim 16, wherein the three-dimensional implantable surgical mesh is a self-fixating mesh including a plurality of grip members extending from the first face, and wherein the plurality of filaments define the plurality of grip members.

18. The three-dimensional composite implant of claim 16, wherein the three-dimensional dry foam is a compressible foam configured to transition from a first foam configuration having a first thickness to a second foam configuration having a second thickness smaller than the first thickness upon the application of pressure thereto, without changing the non-planar configuration of the three-dimensional implantable surgical mesh.

19. The three-dimensional composite implant of claim 16, wherein the three-dimensional implantable surgical mesh is configured to transition to a generally planar implantable surgical mesh when the three-dimensional dry foam is hydrated or absorbed.

20. A method of soft tissue repair comprising:
inserting a three-dimensional composite implant into a site of implantation of a patient near a wound in the soft tissue in need of repair, the three-dimensional composite implant including a mesh having a first face and a second face opposite the first face, a plurality of grip members extending from the first face of the mesh, each of the plurality of grip members including a body extending between a first end attached to the mesh and a second end free of the mesh, and a compressible foam covering at least the second ends of the plurality of grip members, the compressible foam configured to transition from a first foam configuration having a first thickness to a second foam configuration having a second thickness smaller than the first thickness, and the grip members configured to transition from an inactive gripping configuration to a gripping configuration,
deploying the three-dimensional composite implant across the wound in the soft tissue
applying pressure to a portion of the compressible foam to transition from the first foam configuration to the second foam configuration having the second thickness less than the first thickness thereby exposing the second ends of at least some portion of the plurality grip members,
transitioning the grip members from the inactive gripping configuration to the active gripping configuration to fixate the implant to the soft tissue.
